# EUROPÄISCHE PATENTSCHRIFT

(11) **EP 3 131 670 B1**
(45) Veröffentlichungstag und Bekanntmachung des Hinweises auf die Patenterteilung: **25.08.2021**
(21) Anmeldenummer: 15715318.0
(22) Anmeldetag: 16.04.2015
(51) Int. Cl.: B01J 31/02

(54) **VERNETZUNGSKATALYSATOR MIT POLYETHER-STRUKTUREINHEITEN**
CROSS-LINKING CATALYST WITH POLYETHER STRUCTURAL UNITS
CATALYSEUR DE RÉTICULATION À MOTIFS CONSTITUTIFS POLYÉTHER

(30) Priorität: 16.04.2014 EP 14164921
(43) Veröffentlichungstag der Anmeldung: 22.02.2017
(73) Patentinhaber: Sika Technology AG, 6340 Baar (CH)
(72) Erfinder: BURCKHARDT, Urs, CH-8049 Zürich (CH); CANNAS, Rita, CH-8600 Dübendorf (CH)
(74) Vertreter: Sika Patent Attorneys
(86) Internationale Anmeldenummer: PCT/EP2015/058324
(87) Internationale Veröffentlichungsnummer: WO 2015/158859

(56) Entgegenhaltungen:
- WO-A1-03/087204
- DE-B3-102006 039 638
- JP-A- 9 202 710
- US-A1- 2011 287 268
- US-A1- 2011 287 268
- DATABASE CA [Online] CHEMICAL ABSTRACTS SERVICE, COLUMBUS, OHIO, US; 20. Juni 2004 (2004-06-20), GEMBITSKII, P. A. ET AL: "A preparation of derivatives of polyguanidine via thermal alkylation of guanidine derivatives by alkylamines", XP002727166, gefunden im STN Database accession no. 2004:643797 & RU 2 230 734 C1 (OBSHCHESTVO OGRANICHENNOI OTVETSTVENNOST'YU "MEZHDUNARODNYI INSTITUT E) 20. Juni 2004 (2004-06-20)

## Beschreibung

### Technisches Gebiet

Die Erfindung betrifft Katalysatoren für härtbare Zusammensetzungen, insbesondere für Silangruppen-haltige Zusammensetzungen.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über freie oder latente Reaktivgruppen wie beispielsweise Isocyanatgruppen, Epoxidgruppen, Hydroxylgruppen, Aminogruppen oder Silangruppen ablaufen, wobei diese nach einem Mischvorgang, durch Erwärmen oder durch Kontakt mit Feuchtigkeit mit sich selbst oder untereinander reagieren und so die in der Zusammensetzung enthaltenen Aufbaukomponenten kovalent zu einem polymeren Netzwerk verbinden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt wird.

Bei Raumtemperatur härtbare Zusammensetzungen auf Basis von Silangruppen-haltigen Polymeren sind ausgeprägt mit dieser Problemstellung konfrontiert. Silangruppen-haltige Polymere sind dabei insbesondere Polyorganosiloxane, welche gemeinhin als "Silicone" bzw. "Siliconkautschuke" bezeichnet werden, und Silangruppen-haltige organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Dialkylzinn(IV)-carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit.

WO 03/087204 A1 beispielsweise offenbart die Verwendung der Amidinbase 1,8-Diazabicyclo[5.4.0]undec-7-en (DBU) als Katalysator für zweikomponentige, schäumbare, Silangruppen-haltige Zusammensetzungen. DE10 2006 039638 A offenbart die Verwendung starker Basen wie DBU oder Guanidinen zur Modifikation von pyrolytisch hergestellten Kieselsäuren mit Organosilanen.

US 2011/287268 A1 als weiteres Beispiel offenbart

Siliconzusammensetzungen, welche als Polykondensationskatalysator Guanidinverbindungen enthalten.

### Darstellung der Erfindung

Aufgabe der vorliegenden Erfindung ist es daher, einen Katalysator für die Vernetzung von härtbaren Zusammensetzungen, insbesondere Silangruppen-haltigen Zusammensetzungen, bereitzustellen, welcher eine hohe katalytische Aktivität für die Vernetzungsreaktion besitzt und damit eine rasche Aushärtung der applizierten Zusammensetzung ermöglicht, sowie eine hohe Selektivität für diese Vernetzungsreaktion aufweist und somit die Lagerstabilität der Zusammensetzung nicht über Gebühr beeinträchtigt. Weiterhin soll der Katalysator einen geringen Dampfdruck und eine hohe Verträglichkeit mit der Zusammensetzung aufweisen, so dass er weder zu Separierung oder Migration noch zum Verdampfen neigt, soll möglichst geruchlos und wenig toxisch sein und soll bei Raumtemperatur flüssig sein, so dass er auch ohne Lösemittel eingesetzt werden kann.

Diese Aufgabe wird durch einen Katalysator nach Anspruch 1 gelöst. Der Katalysator nach Anspruch 1 enthält aliphatische Amidin- oder Guanidin-Gruppen und zeigt eine hohe katalytische Aktivität, während aromatische Amidine oder Guanidine kaum oder gar nicht katalytisch aktiv sind. Im Gegensatz zu vielen aus dem Stand der Technik bekannten aliphatischen Amidin- oder Guanidin-Katalysatoren ist er bei Raumtemperatur geruchlos, flüssig und überraschend niedrigviskos. Er zeigt eine sehr hohe kalatytische Aktivität bei guter Selektivität, insbesondere in Silangruppen-haltigen Zusammensetzungen. Dies ist besonders überraschend, würde man doch aufgrund seines relativ hohen Molekulargewichtes eine verminderte Aktivität im Vergleich mit kleinen und somit mobileren Amidinen oder Guanidinen erwarten.

Mit diesen Eigenschaften ist der Katalysator nach Anspruch 1 hervorragend geeignet für die Verwendung in Silangruppen-haltigen Zusammensetzungen, wo er als alleiniger Katalysator oder in Kombination mit weiteren Katalysatoren eine schnelle Aushärtung zu einem mechanisch hochwertigen und beständigen Material ermöglicht, ohne die Lagerfähigkeit der unausgehärteten Zusammensetzung zu beeinträchtigen. Überraschenderweise ist er sowohl vor als auch nach der Aushärtung ausgezeichnet verträglich mit härtbaren Zusammensetzungen und neigt weder zu Separierung noch zu Migration. Besonders überraschend ist dabei, dass der Katalysator bei der Verwendung als Vernetzungskatalysator in härtbaren Zusammensetzungen bei deren Aushärtung nicht an die Oberfläche migriert, obwohl er keine funktionellen Gruppen, wie beispielsweise Silangruppen, enthält, die einen Einbau ins ausgehärtete Polymer ermöglichen würden. Er ermöglicht emissions- und geruchsarme Produkte, welche weder schmierige oder klebrige Oberflächen aufweisen noch Substratverschmutzung verursachen, obwohl der Katalysator bei der Aushärtung typischerweise nicht ans Polymer angebunden wird. Schliesslich ist der Katalysator nach Anspruch 1 in einem überraschend einfachen und schnellen Verfahren ohne Hilfsstoffe aus handelsüblichen, preisgünstigen Ausgangsmaterialien herstellbar.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Katalysator der Formel (I),

A⁅+Z]ₙ (I)

wobei
n für 1 oder 2 oder 3 steht,
A für einen Polyoxyalkylen-Rest aus der Polyaddition von Oxiranen, insbesondere Ethylenoxid und/oder 1,2-Propylenoxid und/oder 1,2-Butylenoxid an Startermoleküle mit zwei aktiven Wasserstoffatomen, insbesondere Wasser, Glykole wie 1,2-Ethandiol, 1,2- oder 1,3-Propandiol, 2-Methyl-1,3-propandiol, 1,2- oder 1,3- oder 1,4-Butandiol, 1,3- oder 1,5-Pentandiol, 3-Methyl-1,5-pentandiol, 1,2- oder 2,5- oder 1,6-Hexandiol, Neopentylglykol, Diethylenglykol, Triethylenglykol, Polyethylenglykole, Dipropylenglykol, Tripropylenglykol, Polypropylenglykole oder Poly(tetramethylenether)glykole oder ein Poly(oxytetramethylen)-Rest aus der ringöffnenden Polymerisation von Tetrahydrofuran, welcher gebenenfalls Anteile anderer Oxyalkylen-Einheiten wie insbesondere 1,2-Oxy-propylen- oder Oxybutylen-Einheiten aufweist; oder für einen Rest einer polyoxyalkylierten Verbindung aus der Polyaddition von Ethylenoxid und/oder 1,2-Propylenoxid und/oder 1,2-Butylenoxid an Startermoleküle mit einem bis drei aktiven Wasserstoffatomen ausgewählt aus Methanol, Ethanol, Isopropanol, Butanol, 2-Ethylhexanol, Laurylalkohol, Stearylalkohol, Fettalkohol-Gemischen aus natürlichen Fetten, Phenol, Alkylphenole, Diolen, Diphenolen und Triolen, wobei A ein Molekulargewicht im Bereich vom 200 bis 5000 g/mol aufweist, steht und gegebenenfalls eine oder zwei endständige primäre oder sekundäre Aminogruppen oder Hydroxylgruppen aufweist, und
Z für einen Rest der Formel 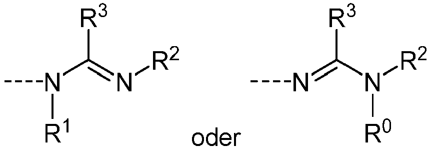 steht,
wobei
R¹ und R⁰ unabhängig voneinander jeweils für Wasserstoff oder für einen Alkyl- oder Cycloalkyl- oder Aralkyl-Rest mit 1 bis 8 C-Atomen stehen,
R² für Wasserstoff oder für einen Alkyl-, Cycloalkyl- oder Aralkyl-Rest mit 1 bis 18 C-Atomen, welcher gegebenenfalls Heteroatome enthält und welcher gegebenenfalls endständige primäre oder sekundäre Aminogruppen aufweist, steht,
R³ für -NR⁴R⁵ oder für Wasserstoff oder für einen Alkyl-, Cycloalkyl- oder Aralkyl-Rest mit 1 bis 12 C-Atomen steht,
R⁴ und R⁵ unabhängig voneinander jeweils für Wasserstoff oder für einen Alkyl-, Cycloalkyl- oder Aralkyl-Rest mit 1 bis 18 C-Atomen, welcher gegebenenfalls Heteroatome enthält, stehen,
wobei
R¹ und R² auch zusammen für einen Alkylen-Rest mit 2 bis 6 C-Atomen stehen können,
R² und R⁰ auch zusammen für einen Alkylen-Rest mit 3 bis 6 C-Atomen, welcher gegebenenfalls Heteroatome enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Heteroatome enthält, stehen können, und
R² und R⁵ auch zusammen für einen Alkylen-Rest mit 2 bis 12 C-Atomen stehen können;
   wobei der Katalysator der Formel (I) kein Stickstoffatom enthält, welches direkt an einen aromatischen Ring gebunden oder Teil eines heteroaromatischen Ringsystems, wie zum Beispiel Imidazol oder Pyrimidin, ist.

Im vorliegenden Dokument bezeichnet der Begriff "Silangruppe" eine an einen organischen Rest oder an einen Polyorganosiloxan-Rest gebundene Silylgruppe mit einer bis drei, insbesondere zwei oder drei, hydrolysierbaren Substituenten am Silicium-Atom. Die hydrolysierbaren Substituenten stellen dabei insbesondere Alkoxy-, Acetoxy-, Ketoximato-, Amido- oder Enoxy-Reste mit 1 bis 13 Kohlenstoffatomen dar, bevorzugt Alkoxy- oder Ketoximato-Reste, besonders bevorzugt Alkoxy-Reste; letztere Silangruppen werden im folgenden als "Alkoxysilangruppen" bezeichnet. Unter dem Begriff "Silangruppen" werden weiterhin Silanolgruppen verstanden, welche formal partiell oder vollständig hydrolysierten Silangruppen entsprechen und einen oder mehrere Hydroxyl-Reste am Siliciumatom aufweisen.
Als "Hydroxysilan", "Isocyanatosilan", "Aminosilan" bzw. "Mercaptosilan" werden Organoalkoxysilane bezeichnet, die am organischen Rest zusätzlich zur Silangruppe eine oder mehrere Hydroxyl-, Isocyanato-, Amino- bzw. Mercaptogruppen aufweisen.
Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.
Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polydiorganosiloxan-Rückgrat (gemeinhin als "Silicone" bezeichnet) stellen keine organischen Polymere im Sinne des vorliegenden Dokuments dar.
Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können. Solche Silangruppen-haltige Polyether können auch als "Silangruppen-haltige Polyurethane" bezeichnet werden.
Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.
Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.
Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Der Katalysator der Formel (I) kann auch in tautomerer Form vorliegen. Alle möglichen Tautomerformen des Katalysators der Formel (I) werden im Rahmen der vorliegenden Erfindung als gleichwertig angesehen.
Weiterhin kann der Katalysator der Formel (I) in protonierter Form vorliegen. Ebenfalls kann der Katalysator der Formel (I) in komplexierter Form vorliegen, insbesondere mit Kationen von Zink, Eisen oder Molybdän.

Im Katalysator der Formel (I) weist A ein mittleres Molekulargewicht im Bereich von 200 bis 5'000 g/mol, besonders bevorzugt 200 bis 2'000 g/mol, auf. Diese Katalysatoren der Formel (I) sind besonders gut zugänglich und niedrigviskos.

Ein als A geeigneter Polyoxyalkylen-Rest ist ein Polyoxyalkylen-Rest aus der Polyaddition von Oxiranen, insbesondere Ethylenoxid und/oder 1,2-Propylenoxid und/oder 1,2-Butylenoxid an Startermoleküle mit zwei aktiven Wasserstoffatomen, insbesondere Wasser, Glykole wie 1,2-Ethandiol, 1,2- oder 1,3-Propandiol, 2-Methyl-1,3-propandiol, 1,2- oder 1,3- oder 1,4-Butandiol, 1,3- oder 1,5-Pentandiol, 3-Methyl-1,5-pentandiol, 1,2- oder 2,5- oder 1,6-Hexandiol, Neopentylglykol, Diethylenglykol, Triethylenglykol, Polyethylenglykole, Dipropylenglykol, Tripropylenglykol, Polypropylenglykole oder Poly(tetramethylenether)glykole.
Ein weiterer als A geeigneter Polyoxyalkylen-Rest ist ein Poly(oxytetramethylen)-Rest aus der ringöffnenden Polymerisation von Tetrahydrofuran, welcher gebenenfalls Anteile anderer Oxyalkylen-Einheiten wie insbesondere 1,2-Oxypropylen- oder Oxybutylen-Einheiten aufweist.
Ein als A geeigneter Rest einer polyoxyalkylierten Verbindung ist ein Rest aus der Polyaddition von Ethylenoxid und/oder 1,2-Propylenoxid und/oder 1,2-Butylenoxid an Startermoleküle mit einem bis drei aktiven Wasserstoffatomen, insbesondere an Alkohole wie Methanol, Ethanol, Isopropanol, Butanol, 2-Ethylhexanol, Laurylalkohol, Stearylalkohol oder Fettalkohol-Gemische aus natürlichen Fetten wie C₁₂₋₁₄-Fettalkohole, Phenol oder Alkylphenole wie Kresol, tert.Butylphenol, Nonylphenol oder Dodecylphenol, an Diole wie 1,4-Dimethylolcyclohexan oder 3(4),8(9)-Bis(hydroxymethyl)tricyclo-[5.2.1.0^{2,6}]decan, an Diphenole wie Bisphenol F oder Bisphenol A oder an Triole wie Glycerin oder 1,1,1-Trimethylolpropan.

Besonders bevorzugt steht A für einen Polyoxypropylen-Rest, gegebenenfalls mit Anteilen anderer Oxyalkylen-Einheiten wie insbesondere Oxyethylen- oder 1,2-Oxybutylen-Einheiten, oder für einen Poly(oxytetramethylen)-Rest, gebenenfalls mit Anteilen von 1,2-Oxypropylen-Einheiten, oder für einen ein- bis dreiwertigen Rest einer polyoxyalkylierten Verbindung aus der Polyaddition von 1,2-Propylenoxid an Startermoleküle ausgewählt aus der Gruppe bestehend aus Alkoholen, Fettalkoholen, Alkylphenolen, Diolen und Triolen. Diese Katalysatoren der Formel (I) sind besonders gut zugänglich und sind besonders gut verträglich mit härtbaren Zusammensetzungen.
Am meisten bevorzugt steht A für einen Polyoxypropylen-Rest mit einem mittleren Molekulargewicht im Bereich von 200 bis 2'000 g/mol.

Bevorzugt ist A weitgehend frei von primären oder sekundären Aminogruppen und/oder von Hydroxylgruppen.

n steht bevorzugt für 1 oder 2, insbesondere für 2.
R¹ und R⁰ stehen bevorzugt unabhängig voneinander jeweils für Wasserstoff oder für einen Alkyl-Rest mit 1 bis 4 C-Atomen, insbesondere für Wasserstoff. R² steht bevorzugt für Wasserstoff oder für einen Alkyl-, Cycloalkyl- oder Aralkyl-Rest mit 1 bis 12 C-Atomen, insbesondere 1 bis 8 C-Atomen, welcher gegebenenfalls Heteroatome enthält.
R³ steht bevorzugt für -NR⁴R⁵ oder für Wasserstoff oder für einen Alkyl-, Cycloalkyl- oder Aralkyl-Rest mit 1 bis 8 C-Atomen.
Besonders bevorzugt steht R³ für -NR⁴R⁵.
R⁴ und R⁵ stehen bevorzugt unabhängig voneinander jeweils für Wasserstoff oder für einen Alkyl-, Cycloalkyl- oder Aralkyl-Rest mit 1 bis 12 C-Atomen, welcher gegebenenfalls ein Sauerstoffatom oder ein Stickstoffatom enthält. Weiterhin bevorzugt stehen R⁴ und R⁵ zusammen für einen Alkylen-Rest mit 4 bis 7 C-Atomen, welcher gegebenenfalls ein Sauerstoffatom oder ein Stickstoffatom enthält.

Weiterhin bevorzugt stehen R¹ und R² zusammen für einen Alkylen-Rest mit 2 bis 4 C-Atomen, insbesondere 2 oder 3 C-Atomen.
Weiterhin bevorzugt stehen R² und R⁰ zusammen für einen Alkylen-Rest mit 4 bis 6 C-Atomen, welcher gegebenenfalls Heteroatome enthält.
Weiterhin bevorzugt stehen R² und R³ zusammen für einen Alkylen-Rest mit 3 bis 5 C-Atomen.
Weiterhin bevorzugt stehen R² und R⁵ zusammen für einen Alkylen-Rest mit 2 bis 4 C-Atomen.
Besonders bevorzugt steht R⁴ für Wasserstoff.

Die bevorzugten Ausführungsformen des Katalysators der Formel (I) lassen sich aus handelsüblichen, preisgünstigen Rohstoffen herstellen und haben besonders gute Eigenschaften in Bezug auf die katalytische Aktivität und Verträglichkeit in härtbaren Zusammensetzungen, insbesondere Silangruppen-haltigen Zusammensetzungen.

In einer Ausführungsform der Erfindung steht im Katalysator der Formel (I) R³ für Wasserstoff oder für einen Alkyl-, Cycloalkyl- oder Aralkyl-Rest mit 1 bis 12, bevorzugt 1 bis 8, insbesondere 1 bis 4, C-Atomen, oder R² und R³ stehen zusammen für einen Alkylen-Rest mit 3 bis 6, insbesondere 3 bis 5, C-Atomen. Diese Katalysatoren der Formel (I) weisen Amidingruppen auf.
Bevorzugt steht R³ für Wasserstoff oder für Methyl, insbesondere für Methyl. Bevorzugt steht R¹ für Wasserstoff oder zusammen mit R² für 1,2-Ethylen oder 1,3-Propylen.
Bevorzugt steht R² für Hexyl, Cyclohexyl, Benzyl, 2-Ethylhexyl, Octyl, Decyl, Dodecyl oder 2-Methoxyethyl, oder zusammen mit R¹ für 1,2-Ethylen oder 1,3-Propylen.
Die bevorzugten Ausführungsformen der Amidingruppen aufweisenden Katalysatoren der Formel (I) zeichnen sich durch eine gute katalytische Aktivität und eine ausgezeichnete Verträglichkeit in härtbaren Zusammensetzungen aus. Gegenüber Guanidingruppen aufweisenden Katalysatoren der Formel (I) weisen sie den Vorteil auf, dass sie eine nicht ganz so hohe katalytische Aktivität besitzten und somit in etwas höherer Menge eingesetzt werden können, wodurch sie weniger anfällig sind für Störungen durch andere Bestandteile der Zusammensetzung, insbesondere den darin enthaltenen Verunreinigungen.

In einer bevorzugten Ausführungsform der Erfindung steht im Katalysator der Formel (I) R³ für-NR⁴R⁵.
Diese Katalysatoren der Formel (I) weisen Guanidingruppen auf. Sie zeichnen sich durch eine niedrige Viskosität, eine im Vergleich zu den Amidingruppen aufweisenden Katalysatoren noch höheren katalytischen Aktivität und eine ausgezeichnete Verträglichkeit in härtbaren Zusammensetzungen aus.

In einem Guanidingruppen aufweisenden Katalysator der Formel (I) steht
R⁴ bevorzugt für Wasserstoff,
R² und R⁵ stehen bevorzugt unabhängig voneinander jeweils für einen Alkyl-, Cycloalkyl- oder Aralkyl-Rest mit 1 bis 18 C-Atomen, welcher gegebenenfalls Heteroatome enthält, und
R¹ steht bevorzugt für Wasserstoff.

Die bevorzugten Ausführungsformen der Guanidingruppen aufweisenden Katalysatoren der Formel (I) weisen die Formel (Ia) auf.

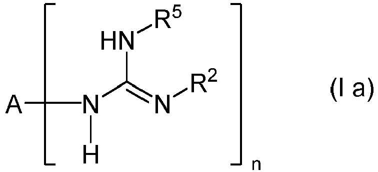

Katalysatoren der Formel (Ia) sind besonders gut herstellbar und weisen eine besonders hohe katalytische Aktivität auf.
R² und R⁵ stehen besonders bevorzugt unabhängig voneinander jeweils für Ethyl, Isopropyl, tert.Butyl, 3-(Dimethylamino)propyl oder Cyclohexyl, insbesondere jeweils für Isopropyl oder Cyclohexyl, am meisten bevorzugt jeweils für Cyclohexyl.
Diese Katalysatoren sind besonders gut zugänglich.

Besonders bevorzugte Guanidingruppen aufweisende Katalysatoren der Formel (I) sind ausgewählt aus der Gruppe bestehend aus 1,1'-(α,ω-Potyoxypropylen)bis(2,3-diisopropylguanidin) mit einem Molekulargewicht im Bereich von etwa 470 bis 2'300 g/mol und 1,1'-(α,ω-Potyoxypropyten)bis(2,3-dicyctohexylguanidin) mit einem Molekulargewicht im Bereich von etwa 630 bis 2'500 g/mol.

Der Katalysator der Formel (I) wird insbesondere erhalten durch die Umsetzung von mindestens einem Polyetheramin mit mindestens einem Reagens zur Einführung einer Amidin- oder Guanidin-Gruppe ausgewählt aus der Gruppe bestehend aus Orthoestern, 1,3-Ketoestern, 1,3-Ketoamiden, Nitrilen, Imidsäureestern, Imidsäurechloriden, Amiden, Lactamen, Cyanamid, Carbodiimiden, Harnstoffen, O-Alkylisoharnstoffen, Thioharnstoffen, S-Alkylisothioharnstoffe, Aminoiminomethansulfonsäuren, Guanylpyrazolen und Guanidinen.

Zur Einführung von Amidingruppen sind Orthoester, 1,3-Ketoester, 1,3-Ketoamide, Nitrile, Imidsäureester, Imidsäurechloride, Amide und Lactame geeignet, insbesondere Orthoester, 1,3-Ketoester und Nitrile.
In einem bevorzugten Verfahren zur Herstellung eines Katalysators der Formel (I), bei welchem R³ für Wasserstoff oder für einen Alkyl-, Cycloalkyl- oder Aralkyl-Rest mit 1 bis 12 C-Atomen steht, wird mindestens ein Polyetheramin mit mindestens einem Orthoester oder mindestens einem 1,3-Ketoester oder mindestens einem Nitril umgesetzt. Dabei entsteht ein Amidingruppen aufweisender Katalysator der Formel (I).
Bevorzugte Orthoester sind Orthoformiate, Orthoacetate, Orthopropionate, Orthobutyrate und Orthovalerate, insbesondere Trimethylorthoformiat, Triethylorthoformiat, Trimethylorthoacetat und Triethylorthoacetat.
Bevorzugte 1,3-Ketoester sind Methylacetoacetat, Ethylacetoacetat, Isopropylacetoacetat oder tert.Butylacetoacetat, insbesondere Ethylacetoacetat. Bevorzugte Nitrile sind Acetonitril, Propionitril, Butyronitril, Isobutyronitril, Valeronitril und Capronitril, insbesondere Acetonitril.

Bevorzugt wird ein Amidingruppen aufweisender Katalysator der Formel (I) erhalten durch die Umsetzung von mindestens einem Polyetheramin mit mindestens einem Orthoester der Formel R³-C(OR^{a})₃ und gegebenenfalls mindestens einem Monoamin der Formel R²-NH-R⁰ , insbesondere R²-NH₂, unter Freisetzung von Alkohol R^{a}OH, wobei R^{a} insbesondere für einen Alkylrest mit 1 bis 4 C-Atomen steht. Diese Umsetzung wird bevorzugt bei erhöhter Temperatur, insbesondere bei 40 bis 160 °C und besonders bevorzugt bei 60 bis 140 °C, und erhöhtem Druck in Gegenwart eines Katalysators durchgeführt.
Weiterhin bevorzugt wird ein Amidingruppen aufweisender Katalysator der Formel (I) erhalten durch die Umsetzung von mindestens einem Polyetheramin mit mindestens einem 1,3-Ketoester der Formel R³-C(O)CH₂C(O)OR^{a}. Diese Umsetzung wird bevorzugt bei einer Temperatur von 20 bis 100 °C, insbesondere 40 bis 80 °C, durchgeführt, wobei der freigesetzte Ester CH₃C(O)OR^{a} bevorzugt destillativ entfernt wird. Bevorzugt wird dabei ein Katalysator eingesetzt, insbesondere eine Säure, bevorzugt eine Sulfonsäure.
Dafür bevorzugte Monoamine sind n-Hexylamin, Cyclohexylamin, Benzylamin, 2-Ethylhexylamin, n-Octylamin, n-Decylamin, Laurylamin und 2-Methoxyethylamin.
Die Umsetzung kann einstufig oder mehrstufig erfolgen.
Das Reaktionsprodukt aus diesem Verfahren wird bevorzugt ohne Aufarbeitung und/oder Reinigung in der Zusammensetzung verwendet, mit Ausnahme der destillativen Entfernung von flüchtigen Verbindungen, gegebenenfalls unter Vakuum.
Beim Einsatz von Polyetherdi- oder -triaminen entstehen typischerweise Gemische, welche neben dem gewünschten Katalysator der Formel (I) oligomere Nebenprodukte mit mehreren Amidingruppen enthalten.
Die Umsetzung erfolgt dann bevorzugt so, dass auf 1 mol Orthoester oder 1,3-Ketoester mindestens 2 mol Polyetherdiamin bzw. 3 mol Polyethertriamin eingesetzt werden, um die Nebenproduktbildung gering zu halten. Die dabei erhaltenen Katalysatoren der Formel (I) weisen primäre oder sekundäre Aminogruppen auf.
Werden neben Polyetherdi- oder -triaminen zusätzlich Monoamine eingesetzt, erhält man ebenfalls weniger oligomere Nebenprodukte. Die Umsetzung erfolgt in diesem Fall bevorzugt so, dass auf 1 mol Polyetherdiamin mindestens 2 mol - bzw. auf 1 mol Polyethertriamin mindestens 3 mol - Monoamin der Formel und mindestens 1 mol Orthoester oder 1,3-Ketoester eingesetzt werden. Die dabei erhaltenen Katalysatoren der Formel (I) sind frei von primären oder sekundären Aminogruppen.
Beim Einsatz von aminopropylierten Polyetheraminen erfolgt die Umsetzung bevorzugt so, dass auf jede Aminopropylaminogruppe ungefähr 1 mol Orthoester oder 1,3-Ketoester eingesetzt wird. Dabei wird ein Katalysator der Formel (I) mit mindestens einer Amidingruppe erhalten, bei welcher R¹ und R² zusammen für 1,3-Propylen stehen. In der folgenden Formel ist ein Umsetzungsprodukt aus Orthoacetat und einem doppelt aminopropylierten Polyoxypropylendiamin im Molverhältnis 2/1 beispielhaft dargestellt:

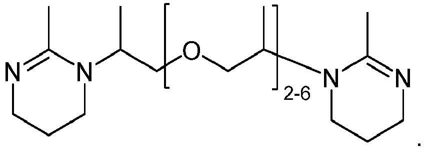

Für den Fall, dass der Katalysator der Formel (I) cyclische Amidingruppen aufweist, ist ein Sechsring aufgrund der hohen katalytischen Aktivität bevorzugt.

Zur Einführung von Guanidingruppen sind Cyanamide, Carbodiimide, Harnstoffe, O-Alkylisoharnstoffe, Thioharnstoffe, S-Alkylisothioharnstoffe, Aminoiminomethansulfonsäuren, Guanylpyrazole und Guanidine geeignet. Bevorzugt sind Cyanamide und Carbodiimide.
Besonders bevorzugt sind Carbodiimide. Auf diesem Weg sind besonders aktive Katalysatoren der Formel (I) auf besonders einfache Weise erhältlich.

In einem weiteren bevorzugten Verfahren zur Herstellung eines Katalysators der Formel (I), bei welchem R³ für -NR⁴R⁵ steht, wird mindestens ein Polyetheramin der Formel A-(NHR¹)ₙ mit mindestens einem Carbodiimid der Formel R⁵-N=C=N-R² umgesetzt. Dabei entsteht insbesondere ein Guanidingruppen aufweisender Katalysator der Formel (Ia). R¹, R², R³, R⁴, R⁵ und n weisen die bereits beschriebenen Bedeutungen auf.

Als Carbodiimid eignen sich typischerweise aliphatische, cycloaliphatische oder arylaliphatische Carbodiimide, insbesondere einfache, im Handel erhältliche aliphatische und cycloaliphatische Carbodiimide, bevorzugt N,N'-Diisopropylcarbodiimid (DIC), N,N'-Di-tert.butylcarbodiimid, N,N'-Dicyclohexylcarbodiimid (DCC) oder N-Ethyl-N'-(3-dimethylaminopropyl)carbodiimid (EDC), besonders bevorzugt N,N'-Diisopropylcarbodiimid (DIC) oder N,N'-Dicyclohexylcarbodiimid (DCC), insbesondere DCC.

Die Umsetzung des Polyetheramins mit dem Carbodiimid wird bevorzugt bei erhöhter Temperatur, insbesondere bei 40 bis 160 °C und besonders bevorzugt bei 60 bis 140 °C durchgeführt. Die Umsetzung kann ganz ohne die Verwendung von VOC-Lösemitteln erfolgen. Zur Beschleunigung der Umsetzung kann ein Katalysator eingesetzt werden, insbesondere eine Säure wie z.B. eine Carbonsäure oder Kohlensäure, oder eine Lewis-Säure wie z.B. Bortrifluorid-Etherat, Aluminiumchlorid, Aluminiumacetylacetonat, Eisen(III)chlorid, Lithiumperchlorat, Zinkchlorid, Zinkacetat, Zink neodecanoat, Zinkacetylacetonat, Zinktriflat oder Lanthantriflat.
Die Umsetzung kann einstufig oder mehrstufig erfolgen.
Bei der Umsetzung wird bevorzugt soviel Carbodiimid eingesetzt, dass pro Equivalent Guanidingruppe, die gebildet werden soll, höchstens ein mol Carbodiimid vorhanden ist.
Insbesondere wird das Carbodiimid stöchiometrisch in Bezug auf die Aminogruppen des Polyetheramins eingesetzt. Auf diese Weise ist der erhaltene Katalysator der Formel (I) weitgehend frei von primären oder sekundären Aminogruppen.
Das Reaktionsprodukt aus diesem Verfahren wird bevorzugt ohne Aufarbeitung und/oder Reinigung verwendet, mit Ausnahme der destillativen Entfernung von flüchtigen Verbindungen, gegebenenfalls unter Vakuum.

Als Polyetheramin für die Herstellung eines Katalysators der Formel (I) geeignet sind Polyoxyalkylene oder polyoxyalkylierte Verbindungen mit endständigen Aminogruppen, wie sie kommerziell beispielsweise unter den Handelsnamen Jeffamine® (von Huntsman), Polyetheramine (von BASF) oder PC Amine® (von Nitroil) erhältlich sind, insbesondere solche mit einem mittleren Molekulargewicht im Bereich von etwa 200 bis 5'000 g/mol, insbesondere die Folgenden:
- Polyetherdiamine mit 2-Aminopropyl- oder 2-Aminobutyl-Endgruppen, insbesondere Jeffamine® D-230, Jeffamine® D-400, Jeffamine® D-2000, Jeffamine® D-4000, Jeffamine® XTJ-582, Jeffamine® XTJ-578, Jeffamine® HK-511, Jeffamine® ED-600, Jeffamine® ED-900, Jeffamine® ED-2003, Jeffamine® XTJ-568, Jeffamine® XTJ-569, Jeffamine® THF-100, Jeffamine® THF-140, Jeffamine® THF-230, Jeffamine® XTJ-533 oder Jeffamine® XTJ-536 (alle von Huntsman).
- Polyetherdiamine mit 4-Aminobutyl-Endgruppen aus der Aminierung von Poly(tetramethylenether)glykolen, insbesondere Jeffamine® THF-170 (von Huntsman).
- Polyethermonoamine, insbesondere Alkohol-gestartete Typen wie Jeffamine® M-600, Jeffamine® M-1000, Jeffamine® M-2005, Jeffamine® M-2070, Jeffamine® XTJ-581, Jeffamine® XTJ-249, Jeffamine® XTJ-435 oder Alkylphenol-gestartete Typen wie Jeffamine® XTJ-436 (alle von Huntsman).
- Polyetherdiamine aus der Polyalkoxylierung von Diolen, insbesondere propoxyliertes 1,4-Dimethylolcyclohexan wie Jeffamine® RFD-270 (von Huntsman).
- Polyethertriamine, insbesondere Jeffamine® T-403, Jeffamine® T-3000, Jeffamine® T-5000 oder Jeffamine® XTJ-566 (alle von Huntsman).
- Polyetheramine mit sekundären Aminogruppen, insbesondere Jeffamine® SD-231, Jeffamine® SD-401, Jeffamine® SD-2001 oder Jeffamine® ST-404 (alle von Huntsman).
- Aminopropylierte Polyetheramine, wie sie durch Umsetzen von Polyetheraminen mit Acrylnitril und nachfolgender Hydrierung erhältlich sind.

Davon bevorzugt sind Polyetheramine mit primären Aminogruppen.

Davon bevorzugt sind weiterhin Polyetheramine mit einem mittleren Molekulargewicht im Bereich von etwa 220 bis 2'000 g/mol.

Ein bevorzugtes Polyetheramin ist ein Polyoxypropylendiamin, welches gegebenenfalls Anteile anderer Oxyalkylen-Einheiten wie insbesondere Oxyethylen- oder 1,2-Oxybutylen-Einheiten aufweist, oder ein Poly(tetramethylenether)diamin, welches gegebenenfalls 1,2-Oxypropylen-Einheiten aufweist, oder ein Polyethermonoamin oder -diamin oder -triamin mit vorwiegend 1,2-Oxypropylen-Einheiten aus der Polyaddition von 1,2-Propylenoxid an Startermoleküle ausgewählt aus der Gruppe bestehend aus Alkoholen, Fettalkoholen, Alkylphenolen, Diolen und Triolen.
Besonders bevorzugt ist das Polyetheramin ein Polyoxypropylendiamin mit einem mittleren Molekulargewicht im Bereich von etwa 220 bis 2'000 g/mol. Am meisten bevorzugt ist das Polyetheramin ein Polyoxypropylendiamin mit einem mittleren Molekulargewicht im Bereich von etwa 400 bis 500 g/mol, insbesondere Jeffamine® D-400 oder Jeffamine® XTJ-582 (beide von Huntsman) oder PC-Amine® DA 400 (von Nitroil) oder Polyetheramine D 400 (von BASF).

In einer bevorzugten Ausführungsform eines Verfahrens zur Herstellung eines Katalysators der Formel (I) wird dieser in Gegenwart eines Silangruppen-haltigen Polymers, hergestellt. Dazu werden das Polyetheramin und das Reagens zur Einführung einer Amidin- oder Guanidin-Gruppe im Silangruppen-haltigen Polymer gelöst oder suspendiert und bei erhöhter Temperatur, insbesondere bei einer Temperatur im Bereich von 40 bis 120 °C, umgesetzt. Diese Herstellung des Katalysators der Formel (I) ist besonders vorteilhaft, da der Katalysator erst mit einer gewissen zeitlichen Verzögerung entsteht, was in der Praxis einen Vorteil darstellen kann.
Diese Herstellungsart ist besonders vorteilhaft für die Umsetzung von Polyetheraminen mit Carbodiimiden.

Der Katalysator der Formel (I) kann insbesondere zur Beschleunigung der Vernetzung von härtbaren Zusammensetzungen eingesetzt werden. Beispiele für geeignete härtbare Zusammensetzungen sind Epoxidharz-Zusammensetzungen, insbesondere heisshärtende, über Dicyandiamid oder Carbonsäuren oder Carbonsäureanhydride vernetzende Systeme, wie sie beispielsweise in Klebstoffen, Beschichtungen und Giessharzen eingesetzt werden; Polyurethan-Zusammensetzungen, insbesondere zweikomponentige, durch Reaktion von Polyolen mit Isocyanaten vernetzende Systeme, wie sie beispielsweise für Klebstoffe, Beläge, Vergussmassen, Dichtfugen, Formkörper oder Blockschäume eingesetzt werden, sowie einkomponentige Systeme mit blockierten Isocyanatgruppen oder blockierten Aminogruppen, wie sie beispielsweise in Pulverlacken, Coil Coatings, Elektrotauchlacken und Flüssiglacken eingesetzt werden; Epoxidharz-Polyurethan-Hybridsysteme; Cyanatesterharz-Zusammensetzungen; sowie Silangruppen-haltige Zusammensetzungen. Besonders vorteilhaft ist die Verwendung in Silangruppen-haltigen Zusammensetzungen, wobei diese auch bei relativ geringer Katalyator-Konzentration rasch aushärten und nicht zu migrationsbedingten Fehlern wie Separation, Ausschwitzen oder Substratverschmutzung neigen.

Dementsprechend betrifft die Erfindung auch die Verwendung eines Katalysators der Formel (I) in einer härtbaren Zusammensetzung, insbesondere einer Silangruppen-haltigen Zusammensetzung, als Vernetzungskatalysator.
Die härtbare Zusammensetzung stellt dabei insbesondere einen Klebstoff, einen Dichtstoff oder eine Beschichtung dar.

Ein weiterer Gegenstand der Erfindung ist eine härtbare Zusammensetzung enthaltend mindestens einen Katalysator der Formel (I) wie vorgängig beschrieben.
Eine solche Zusammensetzung enthält insbesondere mindestens ein Epoxidharz oder mindestens ein Polyisocyanat oder mindestens ein blockiertes Isocyanat oder mindestens ein Cyanesterharz oder mindestens ein Silangruppen-haltiges Polymer.

Bevorzugt enthält die härtbare Zusammensetzung mindestens ein Silangruppen-haltiges Polymer.

Das Silangruppen-haltige Polymer ist in einer Ausführungsform ein Polyorganosiloxan mit endständigen Silangruppen, welches bevorzugt die Formel (III) aufweist, 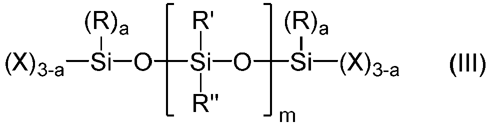 wobei
R, R' und R" unabhängig voneinander jeweils für einen einwertigen Kohlenwasserstoff-Rest mit 1 bis 12 C-Atomen stehen;
X für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.
R' und R" stehen bevorzugt unabhängig voneinander jeweils für einen Alkylrest mit 1 bis 5, bevorzugt 1 bis 3 C-Atomen, insbesondere für Methyl.
X steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht X für einen Hydroxylrest.
a steht bevorzugt für 0 oder 1, insbesondere für 0.
Weiterhin ist m bevorzugt so gewählt, dass das Polyorganosiloxan der Formel (III) bei Raumtemperatur eine Viskosität im Bereich von 100 bis 500'000 mPa·s, insbesondere von 1000 bis 100'000 mPa·s, aufweist.

Solche Polyorganosiloxane sind gut handhabbar und vernetzen mit Feuchtigkeit und/oder Silanvernetzern zu festen Silicon-Polymeren mit elastischen Eigenschaften.

Geeignete kommerziell erhältliche Polyorganosiloxane sind beispielsweise erhältlich von Wacker, Momentive Performance Material, GE Advanced Materials, Dow Corning, Bayer oder Shin Etsu.

Bevorzugt enthält die härtbare Zusammensetzung zusätzlich zum Polyorganosiloxan mit endständigen Silangruppen einen Silanvernetzer, insbesondere ein Silan der Formel (IV),

(R"')_{q}-Si-(X')_{4-q} (IV)

wobei
R"' für einen einwertigen Kohlenwasserstoff-Rest mit 1 bis 12 C-Atomen steht, X' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silane der Formel (IV) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan und Methyltris(isobutylketoximo)silan.

Das Silangruppen-haltige Polymer ist in einer bevorzugten Ausführungsform der Erfindung ein Silangruppen-haltiges organisches Polymer, insbesondere ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden.

Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere. Am meisten bevorzugt ist ein Silangruppen-haltiger Polyether.

Als Silangruppen am organischen Polymer bevorzugt sind Alkoxysilangruppen, insbesondere Endgruppen der Formel (V), 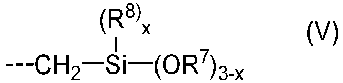 wobei
R⁷ für einen linearen oder verzweigten, einwertigen Kohlenwasserstoffrest mit 1 bis 5 C-Atomen, insbesondere für Methyl oder für Ethyl oder für Isopropyl, steht;
R⁸ für einen linearen oder verzweigten, einwertigen Kohlenwasserstoffrest mit 1 bis 8 C-Atomen, insbesondere für Methyl oder für Ethyl, steht; und
x für einen Wert von 0 oder 1 oder 2, bevorzugt für 0 oder 1, insbesondere für 0, steht.
Besonders bevorzugt steht R⁷ für Methyl oder für Ethyl.
Für bestimmte Anwendungen steht der Rest R⁷ bevorzugt für eine Ethylgruppe, da in diesem Fall bei der Aushärtung der Zusammensetzung ökologisch und toxikologisch harmloses Ethanol freigesetzt wird.
Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8 Silangruppen pro Molekül auf. Die Silangruppen sind bevorzugt endständig.
Das Silangruppen-haltige organische Polymer weist bevorzugt ein mittleres Molekulargewicht, bestimmt mittels GPC gegenüber Polystyrol-Standard, im Bereich von 1000 bis 30'000 g/mol, insbesondere von 2000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.
In einem Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.
In einem weiteren Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.
In einem weiteren Verfahren sind Silangruppen-haltige Polyether erhältlich aus der Umsetzung von Polyetherpolyolen mit Isocyanatosilanen, gegebenenfalls unter Kettenverlängerung mit Diisocyanaten.
In einem weiteren Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.
Bevorzugte Diisocyanate sind ausgewählt aus der Gruppe bestehend aus 1,6-Hexamethylendiisocyanat (HDI), 1-lsocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI). Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyl-trimethoxysilan, 4-Amino-3-methylbutyl-trimethoxysilan, 4-Amino-3,3-dimethylbutyl-trimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)amino-bernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.
Dafür geeignete Aminosilane sind insbesondere 3-Aminopropyl-trimethoxysilan, 3-Aminopropyl-triethoxysilan, 4-Aminobutyl-trimethoxysilan, 4-Aminobutyl-triethoxysilan, 4-Amino-3-methylbutyl-trimethoxysilan, 4-Amino-3-methylbutyl-triethoxysilan, 4-Amino-3,3-dimethylbutyl-trimethoxysilan, 4-Amino-3,3-dimethylbutyl-triethoxysilan, 2-Aminoethyl-trimethoxysilan oder 2-Aminoethyltriethoxysilan. Besonders bevorzugt sind 3-Aminopropyl-trimethoxysilan, 3-Aminopropyl-triethoxysilan, 4-Amino-3,3-dimethylbutyl-trimethoxysilan oder 4-Amino-3,3-dimethylbutyl-triethoxysilan.
Als Lactone geeignet sind insbesondere γ-Valerolacton, γ-Octalacton, δ-Decalacton, und ε-Decalacton, insbesondere γ-Valerolacton.
Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen-haltige Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Bayer MaterialScience AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61 LV, 77, 80, 81); Geniosil® STP (von Wacker Chemie AG; insbesondere die Typen E10, E15, E30, E35).

Besonders bevorzugte Endgruppen der Formel (V) sind Endgruppen der Formel (VI), 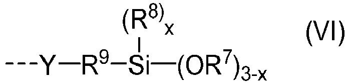 wobei
R⁹ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
Y für einen zweiwertigen Rest ausgewählt aus -O-, -S-, -N(R¹⁰)-, -O-CO-N(R¹⁰)-, -N(R¹⁰)-CO-O- und -N(R¹⁰)-CO-N(R¹⁰)- steht,
   wobei R¹⁰ für Wasserstoff oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilyl-, Ether- oder Carbonsäureestergruppe aufweist, steht; und
R⁷, R⁸ und x die bereits genannten Bedeutungen aufweisen.
Bevorzugt steht R⁹ für 1,3-Propylen oder für 1,4-Butylen, wobei Butylen mit einer oder zwei Methylgruppen substituiert sein kann.
Besonders bevorzugt steht R⁹ für 1,3-Propylen.

Bevorzugt ist der Katalysator der Formel (I) in einer Silangruppen-haltigen Zusammensetzung in einer solchen Menge vorhanden, dass das Gewichtsverhältnis zwischen dem Silangruppen-haltigen Polymer und dem Katalysator der Formel (I) mindestens 10/1, bevorzugt mindestens 20/1, beträgt.
Weiterhin bevorzugt ist der Katalysator der Formel (I) in einer Silangruppen-haltigen Zusammensetzung in einer solchen Menge vorhanden, dass das Gewichtsverhältnis zwischen dem Silangruppen-haltigen Polymer und dem Katalysator der Formel (I) höchstens 5'000/1, bevorzugt höchstens 2'000/1, insbesondere höchstens 1'000/1, beträgt.
Bevorzugt liegt das Gewichtsverhältnis zwischen dem Silangruppen-haltigen Polymer und dem Katalysator der Formel (I) im Bereich von 10/1 bis 2'000/1, insbesondere 20/1 bis 2'000/1.
Eine solche Zusammensetzung weist eine gute Lagerfähigkeit und eine schnelle Aushärtung auf.

Zusätzlich zum Katalysator der Formel (I) kann die härtbare Zusammensetzung weitere Katalysatoren, insbesondere für die Vernetzung von Silangruppen, enthalten. Als weitere Katalysatoren geeignet sind insbesondere Metall-Katalysatoren und/oder basische Stickstoff- oder Phosphorverbindungen.

Mögliche Metall-Katalysatoren sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Mögliche basische Stickstoff- oder Phosphorverbindungen sind insbesondere Imidazole, Pyridine, Phosphazen-Basen oder bevorzugt Amine, Hexahydrotriazine oder Biguanide sowie Amidine oder Guanidine, welche nicht der Formel (I) entsprechen.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen® (von Akzo Nobel) oder Rofamin® (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicyclo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, erhältlich beispielsweise unter dem Handelsnamen Duomeen® (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropyl-terminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethylethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropylmethyldimethoxysilan, N-(2-Aminoethyl)-N'-[3-(trimethoxysilyl)propyl]ethylendiamin oder deren Analoga mit Ethoxy- anstelle der Methoxygruppen am Silicium.
Geeignete Triazine sind insbesondere 1,3,5-Hexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete Amidine, welche nicht der Formel (I) entsprechen, sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.
Geeignete Guanidine, welche nicht der Formel (I) entsprechen, sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)propyl)-1,1,3,3-tetramethylguanidin, 2-(3-(Triethoxysilyl)propyl)-1,1,3,3-tetramethylguanidin, 1,5,7-Triazabicyclo[4.4.0]-dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Weiterhin kann die härtbare Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die härtbare Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

In einer Ausführungsform der Erfindung enthält die härtbare Zusammensetzung neben einem Katalysator der Formel (I) zusätzlich mindestens ein Organotitanat. Eine Kombination aus Katalysator der Formel (I) und Organotitanat weist eine besonders hohe katalytische Aktivität auf. Dadurch wird eine schnelle Aushärtung einer solchen Zusammensetzung bei verhältnismässig geringer Einsatzmenge des Katalysators der Formel (I) ermöglicht.
Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
   wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

In einer Organotitanat-haltigen Zusammensetzung liegt das Gewichtsverhältnis zwischen einem Silangruppen-haltigen Polymer und dem Katalysator der Formel (I) bevorzugt im Bereich von 20/1 bis 2'000/1, insbesondere 40/1 bis 2'000/1.

Das Gewichtsverhältnis zwischen dem Organotitanat und dem Katalysator der Formel (I) liegt bevorzugt im Bereich von 10/1 bis 1/10, besonders bevorzugt 5/1 bis 1/5, insbesondere 5/1 bis 1/3.

Die härtbare Zusammensetzung kann zusätzlich zum Katalysator der Formel (I) weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere trialkylsilylterminierte Polydialkylsiloxane wie insbesondere trimethylsilylterminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen-haltige Polymere in Form von Polyorganosiloxanen;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis-oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
   sowie weitere üblicherweise in härtbaren Zusammensetzungen eingesetzte Substanzen.Es kann sinnvoll sein, gewisse Bestandteile vor dem Einmischen in die Zusammensetzung chemisch oder physikalisch zu trocknen.

In einer bevorzugten Ausführungsform enthält die härtbare Zusammensetzung mindestens ein Trocknungsmittel und mindestens einen Haftvermittler und/oder Vernetzer.

In einer bevorzugten Ausführungsform enthält die härtbare Zusammensetzung keine Phthalate als Weichmacher. Solche Zusammensetzungen sind toxikologisch vorteilhaft und weisen weniger Probleme mit Migrationseffekten auf.

Die härtbare Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.
Die härtbare Zusammensetzung kann in Form einer einkomponentigen oder in Form einer mehrkomponentigen, insbesondere zweikomponentigen, Zusammensetzung vorliegen.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist.
Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Die härtbare Zusammensetzung eignet sich für eine Vielzahl von Verwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.
Besonders geeignet ist die härtbare Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.
Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.
Für eine Anwendung als Klebstoff oder Dichtstoff weist die härtbare Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die härtbare Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Weiterhin betrifft die Erfindung eine ausgehärtete Zusammensetzung, welche erhältlich ist aus einer härtbaren Zusammensetzung enthaltend mindestens einen Katalysator der Formel (I) wie vorgängig beschrieben, nach deren Aushärtung. Die Aushärtung erfolgt insbesondere mit Wasser, insbesondere in Form von Luftfeuchtigkeit, und/oder mit einem geeigneten Vernetzer.

Aus der Verwendung der härtbaren Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

Eine bevorzugte härtbare Zusammensetzung enthält mindestens ein Silangruppen-haltiges Polymer. Eine solche Zusammensetzung wird im Folgenden auch als "silanvernetzende Zusammensetzung" bezeichnet.
Üblicherweise beträgt der Anteil an Silangruppen-haltigem Polymer in einer silanvernetzenden Zusammensetzung 10 bis 80 Gew.-%, insbesondere 15 bis 60 Gew.-%, bevorzugt 15 bis 50 Gew.-%, bezogen auf das Gesamtgewicht der Zusammensetzung.
Für den Fall, dass die silanvernetzende Zusammensetzung ein Polyorganosiloxan mit endständigen Silangruppen enthält, ist sowohl eine einkomponentige Zusammensetzung, auch als RTV-1 bezeichnet, als auch eine zweikomponentige Zusammensetzung, auch als RTV-2 bezeichnet, bevorzugt. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silan der Formel (IV), ist bevorzugt ein Bestandteil der zweiten Komponente. Der Katalysator der Formel (I) kann dabei in der ersten und/oder in der zweiten Komponente enthalten sein.
Für den Fall, dass die silanvernetzende Zusammensetzung ein Silangruppen-haltiges organisches Polymer enthält, ist die Zusammensetzung bevorzugt einkom ponentig.
Eine zweite oder gegebenenfalls weitere Komponenten wird bzw. werden mit der ersten Komponente vor oder bei der Applikation vermischt, insbesondere über einen Statikmischer oder über einen dynamischen Mischer.

Eine silanvernetzende Zusammensetzung wird insbesondere umgebungswarm, bevorzugt in einem Temperaturbereich zwischen 0 und 45 °C, insbesondere 5 bis 35 °C, appliziert und härtet auch bei diesen Bedingungen aus. Bei der Applikation beginnt die Vernetzungsreaktion der Silangruppen, gegebenenfalls unter Einfluss von Feuchtigkeit. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden. Als Ergebnis dieser Reaktionen härtet die Zusammensetzung schliesslich aus. Der Katalysator der Formel (I) beschleunigt diese Aushärtung.
Falls für die Aushärtung Wasser benötigt wird, kann dieses entweder aus der Luft stammen (Luftfeuchtigkeit), oder aber die Zusammensetzung kann mit
einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine
Paste ist insbesondere geeignet für den Fall, dass die Zusammensetzung selber in Form einer Paste vorliegt.
Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die silanvernetzende Zusammensetzung verfügt typischerweise über eine gute Lagerfähigkeit ohne Separationsneigung, erlaubt aufgrund der geringen Toxizität und geringen Flüchtigkeit des Katalysators der Forrmel (I) eine niedrige Gefahreneinstufung und ermöglicht emissions- und geruchsarme Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen enthaltend Katalysatoren nach dem Stand der Technik, wie beispielsweise DBU, TMG oder DHA. Zusammensetzungen enthaltend solche Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Bei der Applikation wird eine silanvernetzende Zusammensetzung bevorzugt auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.
Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die silanvernetzende Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit dem Katalysator der Formel (I) nicht beobachtet.

Besonders geeignet ist die silanvernetzende Zusammensetzung als Klebstoff und/oder Dichtstoff oder als Beschichtung.
Verklebt oder abgedichtet werden können zwei gleichartige oder zwei verschiedene Substrate, insbesondere die vorgängig genannten Substrate.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1 °C und eine relative Luftfeuchtigkeit von 50±5% bezeichnet.

**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹). **Viskositäten** wurden auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 S⁻¹) gemessen.
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.
Die mechanischen Eigenschaften **Zugfestigkeit, Bruchdehnung** und **E-Modul** (bei 0-5% und bei 0-50% Dehnung) wurden gemäss DIN EN 53504 bei einer Zuggeschwindigkeit von 200 mm/min. gemessen.
Die **Shore A-Härte** wurde bestimmt nach DIN 53505 an während 7 Tagen im Normklima ausgehärteten Prüfkörpern.

### Herstellung von Katalysatoren der Formel (I):

**Katalysator K-1:** 1,1'-(α,ω)-Polyoxypropylen)bis(2,3-dicyclohexylguanidin) mit einem Molekulargewicht von etwa 650 g/mol
In einem Rundkolben wurden 5.49 g Polyoxypropylendiamin mit einem mittleren Molekukargewicht von ca. 230 g/mol (Jeffamine® D-230 von Huntsman, Amingehalt ca. 8.4 meq/g) und 8.26 g N,N'-Dicyclohexylcarbodiimid vermischt und die Mischung unter Rühren auf 110 °C erwärmt. In regelmässigen Abständen wurde das Reaktionsgemisch mittels FT-IR-Spektroskopie untersucht. Nach 22 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 13.12 g eines hellgelben, geruchlosen Öls.
FT-IR: 3366 (N-H), 2923, 2950, 1635 (C=N), 1495, 1447, 1370, 1336, 1255, 1237, 1102, 1025, 977, 888, 845, 713.

**Katalysator K-2:** 1,1'-(α,ω)-Polyoxypropylen)bis(2,3-dicyclohexylguanidin) mit einem Molekulargewicht von etwa 850 g/mol
In einem Rundkolben wurden 10.54 g Polyoxypropylendiamin mit einem mittleren Molekukargewicht von ca. 430 g/mol (Jeffamine® D-400 von Huntsman, Amingehalt ca. 4.4 meq/g) und 8.23 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 18.77 g eines hellgelben, geruchlosen Öls mit einer Viskosität bei 25 °C von 12.3 Pa·s.
FT-IR: 3357 (N-H), 2968, 2924, 2951, 1637 (C=N), 1498, 1448, 1372, 1340, 1256, 1237, 1100, 1018, 926, 889, 845, 715.

**Katalysator K-3:** 1-(ω-Methoxy-polyoxypropylen-polyoxyethylen)-2,3-dicyclohexylguanidin mit einem Molekulargewicht von etwa 810 g/mol In einem Rundkolben wurden 14.32 g Polyoxypropylenmonoamin mit einem mittleren Molekukargewicht von ca. 600 g/mol (Jeffamine® M-600 von Huntsman, Amingehalt ca. 1.7 meq/g) und 4.14 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 72 Stunden war die Carbodiimid-Bande bei ca. 2120 cm⁻¹ vollständig verschwunden. Man erhielt 18.45 g eines hellgelben, geruchlosen Öls mit einer Viskosität bei 25 °C von 0.3 Pa·s.
FT-IR: 3367 (N-H), 2969, 2926, 2854, 1640 (C=N), 1449, 1372, 1342, 1297, 1255, 1100, 1014, 925, 889, 861, 715.

**Katalysator K-4:** 1,1'-(α,ω)-Polyoxypropylen)bis(2,3-dicyclohexylguanidin) mit einem Molekulargewicht von etwa 2'400 g/mol
In einem Rundkolben wurden 24.06 g Polyoxypropylendiamin mit einem mittleren Molekukargewicht von ca. 2'000 g/mol (Jeffamine® D-2000 von Huntsman, Amingehalt ca. 1.0 meq/g) und 4.15 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 72 Stunden war die Carbodiimid-Bande bei ca. 2120 cm⁻¹ vollständig verschwunden. Man erhielt 28.21 g eines hellgelben, geruchlosen Öls mit einer Viskosität bei 25 °C von 2.4 Pa·s.
FT-IR: 3367 (N-H), 2969, 2926, 2856, 1641 (C=N), 1449, 1372, 1343, 1296, 1256, 1097, 1013, 925, 866, 833, 715.

**Katalysator K-5:** 1,1'-(α,ω)-Polyoxypropylen)bis(2,3-diisopropylguanidin) mit einem Molekulargewicht von etwa 690 g/mol
In einem Rundkolben wurden 5.57 g Polyoxypropylendiamin mit einem mittleren Molekukargewicht von ca. 430 g/mol (Jeffamine® D-400 von Huntsman, Amingehalt ca. 4.4 meq/g) und 2.95 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 18 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 8.50 g eines hellgelben, geruchlosen Öls mit einer Viskosität bei 25 °C von 0.4 Pa·s.
FT-IR: 3365 (N-H), 2955, 2929, 2967, 1640 (C=N), 1452, 1372, 1338, 1295, 1099, 1018, 923, 859, 845, 713.

**Katalysator K-6:** N,N"-(α,ω-Polyoxypropylen)bis(N'-hexylacetimidamid) mit einem Molekulargewicht von etwa 480 g/mol
In einem Reagensglas wurden 9.63 g Polyoxypropylendiamin mit einem mittleren Molekukargewicht von ca. 230 g/mol (Jeffamine® D-230 von Huntsman, Amingehalt ca. 8.4 meq/g), 8.11 g n-Hexylamin, 10.21 g Trimethylorthoacetat und 0.33 g Lanthantriflat vermischt und in einem Mikrowellengerät bei einem Maximaldruck von 150 Pa unter Rühren während 40 Minuten auf 160 °C erwärmt. Darauf wurde das Reaktionsgemisch in einen Rundkolben transferiert und im Vakuum von den flüchtigen Bestandteilen befreit. Man erhielt 20.24 g eines gelben, geruchlosen Öls.
FT-IR: 3369, 2958, 2926, 2856, 1684, 1644, 1524, 1454, 1373, 1259, 1101, 1031, 926, 725.

### Herstellung von Silangruppen-haltigen Polymeren:

### Polymer STP-1:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)-amino-bernsteinsäurediethylester (Addukt aus 3-Aminopropyltriethoxysilan und Maleinsäurediethylester) eingemischt und die Mischung bei 90 °C solange gerührt, bis mittels FT-IR-Spektroskopie kein freies Isocyanat mehr nachgewiesen wurde. Der so erhaltene Triethoxysilangruppen-haltige Polyether mit einem Silan-Equivalentgewicht von ca. 6920 g/Eq (aus den Einsatzmengen berechnet) wurde auf Raumtemperatur abgekühlt und unter Ausschluss von Feuchtigkeit aufbewahrt.

### Verwendete kommerzielle Katalysatoren und Abkürzungen dafür:

| | |
|---|---|
| DBU | 1,8-Diazabicyclo[5.4.0]undec-7-en |
| TMG | 1,1,3,3-Tetramethylguanidin |
| IBAY | Tyzor® IBAY, Bis(ethylacetoacetato)diisobutoxy-titan(IV), von Dorf Ketal |
| MTHP | 2-Methyl-1,4,5,6-tetrahydropyrimidin |
| TMTHP | 2,5,5-Trimethyl-1,4,5,6-tetrahydropyrimidin |
| DBTDL | Dibutylzinn(IV)dilaurat |

### Silanvernetzende Zusammensetzungen:

Die Zusammensetzungen Z1 bis Z25 sind erfindungsgemässe Beispiele, die Zusammensetzungen V1 bis V26 sind Vergleichsbeispiele.

### Zusammensetzungen Z1 bis Z2b und Vergleiche V1 bis V5:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten gelassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V1** | DBU | 0.15 g | 1.0 | 24.8 | 30.3 | 22% | 37' | 42' |
| **V2** | TMG | 0.11 g | 1.0 | 20.3 | 21.8 | 7% | 93' | 108' |
| **V3** | IBAY | 0.87 g | 2.0 | 24.8 | 30.2 | 22% | 45' | 120' |
| **V4** | DBU | 0.07 g | 0.5 | 24.9 | 28.1 | 13% | 15' | 55' |
| | IBAY | 0.22 g | 0.5 | | | | | |
| **V5** | TMG | 0.06 g | 0.5 | 23.2 | 25.0 | 8% | 37' | 111' |
| | IBAY | 0.22 g | 0.5 | | | | | |
| **Z1** | K-1 | 0.32 g | 1.0 | 21.0 | 23.3 | 11% | 21' | 27' |
| **Z2** | K-1 | 0.16 g | 0.5 | 24.4 | 29.1 | 19% | 17' | 53' |
| | IBAY | 0.22 g | 0.5 | | | | | |
| **Z2a** | K-2 | 0.77 g | 1.9 | 27.8 | 37.4 | 35% | 13' | 12' |
| **Z2b** | K-6 | 0.45 g | 1.9 | 28.6 | 33.3 | 16% | 50' | 50' |

| | | | | | | | | |
|---|---|---|---|---|---|---|---|---|
| ¹ mmol Amidin- oder Guanidin-Gruppen oder Metallatome auf 100 g Silangruppen-haltiges Polymer. ² während 7 Tagen bei 60 °C in geschlossenem Gebinde. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V1** | schmierig, leicht klebrig | 0.60 MPa | 92% | 1.03 MPa | 0.69 MPa |
| **V2** | stark klebrig | 0.65 MPa | 90% | 1.20 MPa | 0.77 MPa |
| **V3** | klebrig | 0.59 MPa | 91% | 1.05 MPa | 0.69 MPa |
| **V4** | leicht schmierig | 0.60 MPa | 81% | 1.11 MPa | 0.76 MPa |
| **V5** | leicht klebrig | 0.69 MPa | 100% | 1.13 MPa | 0.76 MPa |
| **Z1** | trocken | 0.63 MPa | 88% | 1.16 MPa | 0.76 MPa |
| **Z2** | trocken | 0.62 MPa | 87% | 1.16 MPa | 0.76 MPa |
| **Z2a** | trocken | 0.62 MPa | 89% | 1.11 MPa | 0.75 MPa |
| **Z2b** | trocken | 0.66 MPa | 96% | 1.09 MPa | 0.78 MPa |

### Zusammensetzungen Z3 bis Z11 und Vergleiche V6 bis V11:

Eine Zusammensetzung aus 95.9 g Polymer STP-2, 0.4 g Vinyltriethoxysilan und 3.7 g 3-Aminopropyltriethoxysilan wurde mit verschiedenen Katalysatoren in der angegebenen Menge gemäss Tabelle 3 vermengt und die Mischung wie für Zusammensetzung Z1 beschrieben auf Viskosität, Hautbildungszeit (HBZ), Oberflächenbeschaffenheit und mechanische Eigenschaften geprüft. geprüft. Die Ergebnisse sind in Tabelle 3 und 4 wiedergegeben. "Zus." steht für "Zusammensetzung" .

**Tabelle 3:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V6** | DBU | 0.55 g | 3.8 | 48.8 | 58.1 | 19% | 127' | 155' |
| **V7** | TMG | 0.42 g | 3.8 | 44.5 | 53.4 | 20% | >12h | >12h |
| **V8** | MTHP | 0.36 g | 3.8 | 46.5 | 54.0 | 16% | 120' | 110' |
| **V9** | TMTHP³ | 0.36 g | 3.0 | 30.0 | 34.4 | 15% | 220' | 180' |
| **V10** | DBU | 0.22 g | 1.5 | 43.2 | 45.6 | 6% | 200' | 180' |
| | IBAY | 0.82 g | 1.9 | | | | | |
| **V11** | TMG | 0.17 g | 1.5 | 38.2 | 42.8 | 12% | >24h | >24h |
| | IBAY | 0.82 g | 1.9 | | | | | |
| **Z3** | K-1 | 1.20 g | 3.8 | 48.7 | 54.9 | 13% | 60' | 73' |
| **Z4** | K-2 | 1.71 g | 3.8 | 38.8 | 39.5 | 2% | 65' | 80' |
| **Z5** | K-3 | 2.94 g | 3.8 | 32.9 | 35.4 | 8% | 75' | 75' |
| **Z6** | K-4 | 4.26 g | 3.7 | 33.3 | 42.0 | 26% | 78' | 78' |
| **Z7** | K-5 | 1.29 g | 3.7 | 35.1 | 41.2 | 17% | 105' | 80' |
| **Z8** | K-1 | 0.48 g | 1.5 | 42.7 | 44.5 | 4% | 125' | 135' |
| | IBAY | 0.82 g | 1.9 | | | | | |
| **Z9** | K-2 | 0.68 g | 1.5 | 43.8 | 44.0 | 1% | 125' | 135' |
| | IBAY | 0.82 g | 1.9 | | | | | |
| **Z10** | K-3 | 1.16 g | 1.5 | 39.2 | 42.4 | 8% | 120' | 125' |
| | IBAY | 0.82 g | 1.9 | | | | | |
| **Z11** | K-4 | 1.73 g | 1.5 | 39.9 | 43.0 | 8% | 115' | 125' |
| | IBAY | 0.82 g | 1.9 | | | | | |

| | | | | | | | | |
|---|---|---|---|---|---|---|---|---|
| ¹ mmol Amidin- oder Guanidin-Gruppen oder Metallatome auf 100 g Silangruppen-haltiges Polymer. ² während 7 Tagen bei 60 °C in geschlossenem Gebinde. ³ als Lösung (20 Gew.-%) in N-Ethylpyrrolidon. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V6** | schmierig, klebrig | 0.55 MPa | 152% | 0.48 MPa | 0.44 MPa |
| **V7** | sehr stark klebrig | n.b. | n.b. | n.b. | n.b. |
| **V8** | schmierig, klebrig | 0.53 MPa | 165% | 0.52 MPa | 0.39 MPa |
| **V9** | schmierig, klebrig | 0.45 MPa | 80% | 0.71 MPa | 0.54 MPa |
| **V10** | leicht schmierig u. klebrig | 0.66 MPa | 145% | 0.81 MPa | 0.56 MPa |
| **V11** | (flüssig) | n.b. | n.b. | n.b. | n.b. |
| **Z3** | trocken | 0.59 MPa | 128% | 0.79 MPa | 0.55 MPa |
| **Z4** | trocken | 0.51 MPa | 77% | 0.87 MPa | 0.62 MPa |
| **Z5** | trocken | 0.63 MPa | 141% | 0.77 MPa | 0.55 MPa |
| **Z6** | trocken | 0.61 MPa | 134% | 0.74 MPa | 0.55 MPa |
| **Z7** | trocken | 0.59 MPa | 105% | 0.92 MPa | 0.60 MPa |
| **Z8** | trocken | 0.60 MPa | 129% | 0.80 MPa | 0.53 MPa |
| **Z9** | trocken | 0.65 MPa | 137% | 0.84 MPa | 0.57 MPa |
| **Z10** | trocken | 0.62 MPa | 122% | 0.84 MPa | 0.57 MPa |
| **Z11** | trocken | 0.54 MPa | 100% | 0.81 MPa | 0.58 MPa |

| | | | | | |
|---|---|---|---|---|---|
| "n.b. steht für "nicht bestimmt" (nicht messbar) | | | | | |

### Zusammensetzungen Z12 bis Z14 und Vergleiche V12 bis V13:

In einem Planetenmischer wurden 36.2 g Polymer STP-2, 60.2 g gemahlene Kreide (Omyacarb® 5 GU, von Omya), 1.2 g Thixotropierpaste, deren Herstellung nachfolgend beschrieben ist, 1.2 g Vinyltriethoxysilan, 1.2 g 3-Aminopropyltriethoxysilan sowie verschiedene Katalysatoren in der angegebenen Menge gemäss Tabelle 5 vermengt und die Mischung wie für Zusammensetzung Z1 beschrieben auf Viskosität, Hautbildungszeit (HBZ), Oberflächenbeschaffenheit und mechanische Eigenschaften geprüft. Die Ergebnisse sind in Tabelle 5 wiedergegeben. "Zus." steht für "Zusammensetzung".
Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Bayer) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 5:**

| **Zus.** | **Katalysator** | **Menge** | **Konzentration¹** | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **V12** | DBU | 0.40 g | 2.6 | 83' | schmierig | 2.5MPa | 155% | 4.0 | 2.0 |
| **V13** | DBU | 0.20 g | 1.3 | 63' | leicht schmierig | 2.2 MPa | 184% | 3.6 | 1.8 |
| | IBAY | 0.59 g | 1.3 | | | | | | |
| **Z12** | K-2 | 1.22 g | 2.6 | 57' | trocken | 3.1 MPa | 167% | 5.0 | 2.5 |
| **Z13** | K-4 | 3.12 g | 2.6 | 65' | trocken | 2.3MPa | 148% | 3.8 | 1.9 |
| **Z14** | K-2 | 0.61 g | 1.3 | 57' | trocken | 2.2 MPa | 196% | 4.7 | 1.9 |
| | IBAY | 0.59 g | 1.3 | | | | | | |

| | | | | | | | | | |
|---|---|---|---|---|---|---|---|---|---|
| ¹ mmol Amidin- oder Guanidin-Gruppen oder Metallatome auf 100 g Zusammensetzung. | | | | | | | | | |

### Zusammensetzungen Z15 bis Z16 und Vergleiche V14 bis V15:

Eine Zusammensetzung aus 98 g Silangruppen-haltiger Polyether MS Polymer™ S203H (von Kaneka) und 2 g 3-Aminopropyltrimethoxysilan wurde mit verschiedenen Katalysatoren in der angegebenen Menge gemäss Tabelle 6 vermengt und die Mischung wie beschrieben auf Hautbildungszeit (HBZ) und Oberflächenbeschaffenheit nach 7 Tagen im Normklima geprüft. Die Ergebnisse sind in Tabelle 6 wiedergegeben. "Zus." steht für "Zusammensetzung".

**Tabelle 6:**

| **Zus.** | **Katalysator** | **Menge** | **Konzentration¹** | **HBZ** | **Oberfläche nach 7d** |
|---|---|---|---|---|---|
| **V14** | DBU | 3.65 g | 24 | >12h | schmierig und klebrig |
| **V15** | DBTDL | 1.52 g | 2.4 | 71' | trocken |
| **Z15** | K-2 | 11.29 g | 24 | 4h 45' | fast trocken |
| **Z16** | DBTDL K-2 | 1.52 g 1.88 g | 2.4 4 | 54' | trocken |

| | | | | | |
|---|---|---|---|---|---|
| ¹ mmol Amidin- oder Guanidin-Gruppen oder Metallatome auf 100 g Silangruppen-haltiger Polyether. | | | | | |

### Zusammensetzungen Z17 bis Z18 und Vergleiche V16 bis V18:

Eine Zusammensetzung aus 96.5 g Silangruppen-haltiger Polyether TEGOPAC® Bond 150 (von Evonik), 0.5 g Vinyltriethoxysilan und 3.0 g 3-Aminopropyltriethoxysilan wurde mit verschiedenen Katalysatoren in der angegebenen Menge gemäss Tabelle 7 vermengt und die Mischung wie beschrieben auf Hautbildungszeit (HBZ) und Oberflächenbeschaffenheit nach 7 Tagen im Normklima geprüft. Die Ergebnisse sind in Tabelle 7 wiedergegeben. "Zus." steht für "Zusammensetzung".

**Tabelle 7:**

| **Zus.** | **Katalysator** | **Menge** | **Konzentration¹** | **HBZ** | **Oberfläche nach 7d** |
|---|---|---|---|---|---|
| **V16** | DBU | 2.28 g | 15 | 4h 20' | schmierig |
| **V17** | DBTDL | 0.79 g | 1.25 | 1h 45' | trocken |
| **V18** | IBAY | 7.25 g | 15 | 2h | weich und klebrig |
| **V19** | DBU | 4.93 g | 5 | 3h | klebrig |
| | IBAY | 2.40 g | 5 | | |
| **Z17** | K-2 | 6.25 g | 14.7 | 1h | fast trocken |
| **Z18** | K-2 | 2.10 g | 5 | 2h 50' | trocken |
| | IBAY | 2.40 g | 5 | | |

| | | | | | |
|---|---|---|---|---|---|
| ¹ mmol Amidin- oder Guanidin-Gruppen bzw. Metallatome auf 100 g Silangruppen-haltiger Polyether. | | | | | |

### Zusammensetzungen Z19 und Vergleiche V20 bis V22

In einem Rundkolben wurden 71.1 g eines OH-terminierten linearen Polydimethylsiloxans mit einer Viskosität von ca. 50'000 mPas bei 23 °C (Wacker® Silicone Rubber Polymer FD 50, von Wacker) mit 2.6 g Vinyl-tris(methylethylketoximo)silan vermengt und während 15 Minuten unter Vakuum gerührt. In das so erhaltene Polydimethylsiloxan mit Vinyl-bis(methylethylketoximo)silyl-Endgruppen wurden 26.3 g Trimethylsilyl-terminiertes Polydimethylsiloxan (Wacker® AK 100 Siliconöl, von Wacker) eingerührt. Diese Mischung wurde mit verschiedenen Katalysatoren gemäss nachstehender Tabelle 8 vermengt und wie für Zusammensetzung Z1 beschrieben auf Viskosität, Hautbildungszeit (HBZ), Oberflächenbeschaffenheit sowie mechanische Eigenschaften geprüft.

Die Ergebnisse sind in Tabelle 8 und 9 wiedergegeben. "Zus." steht für "Zusammensetzung".

**Tabelle 8:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V20** | DBU | 0.04 g | 0.4 | 18.2 | 15.9 | -13% | 10' | 12' |
| **V21** | TMG | 0.03 g | 0.4 | 17.4 | 16.5 | -5% | 23' | 27' |
| **V22** | IBAY | 0.13 g | 0.4 | 19.7 | 19.9 | 1% | 27' | 47' |
| **Z19** | K-2 | 0.13 g | 0.4 | 17.9 | 16.2 | -10% | 14' | 16' |

| | | | | | | | | |
|---|---|---|---|---|---|---|---|---|
| ¹ mmol Amidin- oder Guanidin-Gruppen oder Metallatome auf 100 g Ketoximato-Polydimethylsiloxan Polymer. ² während 7 Tagen bei 70 °C in geschlossenem Gebinde. | | | | | | | | |

**Tabelle 9:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V20** | trocken | 0.22 MPa | 135% | 0.17 MPa | 0.13 MPa |
| **V21** | trocken | 0.15 MPa | 86% | 0.17 MPa | 0.16 MPa |
| **V22** | trocken | 0.25 MPa | 116% | 0.17 MPa | 0.18 MPa |
| **Z19** | trocken | 0.26 MPa | 282% | 0.19 MPa | 0.17 MPa |

### Zusammensetzungen Z20 bis Z21 und Vergleiche V23 bis V24

In einem Kunststoffbecher wurden 20.2 g einer ersten Komponente bestehend aus 99 Gewichtsteilen (GT) eines OH-terminierten linearen Polydimethylsiloxans mit einer Viskosität von ca. 50'000 mPas bei 23 °C (Wacker® Silicone Rubber Polymer FD 50, von Wacker) und 1 GT Wacker® E 2 Siliconöl-Emulsion (nichtionisch in Wasser emulgiertes mittelviskoses OH-terminiertes lineares Polydimethylsiloxan, von Wacker; Feststoffgehalt 37-40%) mit einer zweiten Komponente bestehend aus 0.80 g Vinyltrimethoxysilan und einem Katalysator in der in der Tabelle 10 angegebenen Art und Menge innig vermischt und die Mischung wie für Zusammensetzung Z1 beschrieben auf Hautbildungszeit (HBZ) und Oberflächenbeschaffenheit geprüft. Weiterhin wurde die applizierte Mischung nach 7 Tagen im Normklima auf Shore A-Härte geprüft. Die Ergebnisse sind in Tabelle 10 wiedergegeben. "Zus." steht für "Zusammensetzung".

**Tabelle 10:**

| **Zus.** | **Katalysator** | **Menge** | **Konzentration ¹** | **HBZ** | **Oberfläche nach 24h** | **Shore A nach 7 d** |
|---|---|---|---|---|---|---|
| **V23** | DBU | 0.06 g | 2.0 | 55' | trocken | 3 |
| **V24** | IBAY | 0.48 g | 5.0 | 40' | trocken | <2 |
| **Z20** | K-2 | 0.17 g | 2.0 | 12' | trocken | 6 |
| **Z21** | K-5 | 0.14 g | 2.0 | 30' | trocken | 5 |

| | | | | | | |
|---|---|---|---|---|---|---|
| ¹ mmol Amidin- oder Guanidin-Gruppen oder Metallatome auf 100 g Zusammensetzung. | | | | | | |

### Zusammensetzung Z22 (in situ-Herstellung des Katalysators):

Eine Zusammensetzung aus 30.0 g Polymer STP-1, 0.15 g Vinyltrimethoxysilan und 1.2 g 3-Glycidoxypropyltrimethoxysilan wurde unter Feuchtigkeitsausschluss mit 0.8 g Polyoxypropylendiamin mit einem mittleren Molekukargewicht von ca. 430 g/mol (Jeffamine® D-400 von Huntsman, Amingehalt ca. 4.4 meq/g) und 0.4 g N,N'-Diisopropylcarbodiimid vermengt, die Mischung in eine innenlackierte Aluminiumtube abgefüllt und im Ofen auf 80 °C erwärmt. Nach den in der Tabelle 11 angegebenen Zeitabständen wurde die Mischung auf Hautbildungszeit (HBZ) im Normklima sowie auf den Umsatz des Carbodiimids (via Abnahme der Intensität der Carbodiimid-Bande bei ca. 2120 cm⁻¹ im FT-IR, Intensität zu Beginn = 0% Umsatz, keine Bande mehr nachweisbar = 100% Umsatz) geprüft. Die Ergebnisse sind in Tabelle 11 wiedergegeben.

**Tabelle 11:**

| **Zeit** | **HBZ** | **Carbodiimid-Umsatz** |
|---|---|---|
| 0h | > 9h | 0% |
| 2h | 3h 05' | 21% |
| 4h | 1h 05' | 39% |
| 6h | 52' | 64% |
| 26h | 43' | 100% |

### Zusammensetzung Z23 (in situ-Herstellung des Katalysators):

Die in situ-Herstellung des Katalysators aus Zusammensetzung Z22 wurde wiederholt, wobei aber das 3-Glycidoxypropyltrimethoxysilan weggelassen wurde . Die Ergebnisse sind in Tabelle 12 wiedergegeben.

**Tabelle 12:**

| **Zeit** | **HBZ** | **Carbodiimid-Umsatz** |
|---|---|---|
| 0h | 7h | 0% |
| 2h | 2h 20' | 20% |
| 4h | 50' | 51% |
| 6h | 32' | 69% |
| 26h | 26' | 100% |

### Zusammensetzungen Z24 bis Z25 und Vergleiche V25 und V26:

Eine Zusammensetzung aus 96.0 g Silangruppen-haltigem Polyether, entweder GENIOSIL® STP-E 15 (von Wacker) oder Desmoseal® S XP 2821 (von Bayer), 0.35 g Vinyltrimethoxysilan und 3.72 g 3-Aminopropyltrimethoxysilan wurde mit verschiedenen Katalysatoren in der angegebenen Menge gemäss Tabelle 13 vermengt und die Mischung wie für Zusammensetzung Z1 beschrieben geprüft. Die Ergebnisse sind in Tabelle 13 wiedergegeben. "Zus." steht für "Zusammensetzung".

**Tabelle 13:**

| **Zus.** | **Polymer** | **Katalysator, Menge** | **Konzentration¹** | **HBZ** | **Oberfläche nach 24h** | **Zugfestigkeit** | **Bruchdehnung** | **E-Modul 0-5%** |
|---|---|---|---|---|---|---|---|---|
| **V25** | E-15² | DBU, 0.28 g | 1.8 | 60' | schmierig | 0.72 MPa | 48% | 1.84 MPa |
| **Z24** | E-15² | K-2, 0.78 g | 1.7 | 13' | trocken | 0.79 MPa | 54% | 1.83 MPa |
| **V26** | 2821³ | DBU, 0.28 g | 1.8 | 55' | trocken | 0.85 MPa | 28% | 3.2 MPa |
| **Z25** | 2821³ | K-2, 0.78 g | 1.7 | 40' | trocken | 0.92 MPa | 29% | 3.2 MPa |

| | | | | | | | | |
|---|---|---|---|---|---|---|---|---|
| ¹ mmol Amidin- oder Guanidin-Gruppen auf 100 g Silangruppen-haltiger Polyether. ² GENIOSIL® STP-E 15 (von Wacker); ³ Desmoseal® S XP 2821 (von Bayer) | | | | | | | | |

## Patentansprüche

1. Katalysator der Formel (I),
A-[-Z]ₙ (I)
wobei
n für 1 oder 2 oder 3 steht,
A für einen Polyoxyalkylen-Rest aus der Polyaddition von Oxiranen, insbesondere Ethylenoxid und/oder 1,2-Propylenoxid und/oder 1,2-Butylenoxid an Startermoleküle mit zwei aktiven Wasserstoffatomen, insbesondere Wasser, Glykole wie 1,2-Ethandiol, 1,2- oder 1,3-Propandiol, 2-Methyl-1,3-propandiol, 1,2- oder 1,3- oder 1,4-Butandiol, 1,3- oder 1,5-Pentandiol, 3-Methyl-1,5-pentandiol, 1,2- oder 2,5- oder 1,6-Hexandiol, Neopentylglykol, Diethylenglykol, Triethylenglykol, Polyethylenglykole, Dipropylenglykol, Tripropylenglykol, Polypropylenglykole oder Poly(tetramethylenether)glykole oder ein Poly(oxytetramethylen)-Rest aus der ringöffnenden Polymerisation von Tetrahydrofuran, welcher gebenenfalls Anteile anderer Oxyalkylen-Einheiten wie insbesondere 1,2-Oxy-propylen- oder Oxybutylen-Einheiten aufweist; oder für einen Rest einer polyoxyalkylierten Verbindung aus der Polyaddition von Ethylenoxid und/oder 1,2-Propylenoxid und/oder 1,2-Butylenoxid an Startermoleküle mit einem bis drei aktiven Wasserstoffatomen ausgewählt aus Methanol, Ethanol, Isopropanol, Butanol, 2-Ethylhexanol, Laurylalkohol, Stearylalkohol, Fettalkohol-Gemischen aus natürlichen Fetten, Phenol, Alkylphenole, Diolen, Diphenolen und Triolen, wobei A ein Molekulargewicht im Bereich vom 200 bis 5000 g/mol aufweist,
steht und gegebenenfalls eine oder zwei endständige primäre oder sekundäre Aminogruppen oder Hydroxylgruppen aufweist, und
Z für einen Rest der Formel 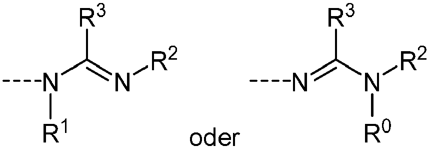 steht,
wobei
R¹ und R⁰ unabhängig voneinander jeweils für Wasserstoff oder für einen Alkyl- oder Cycloalkyl- oder Aralkyl-Rest mit 1 bis 8 C-Atomen stehen,
R² für Wasserstoff oder für einen Alkyl-, Cycloalkyl- oder Aralkyl-Rest mit 1 bis 18 C-Atomen, welcher gegebenenfalls Heteroatome enthält und welcher gegebenenfalls endständige primäre oder sekundäre Aminogruppen aufweist, steht,
R³ für -NR⁴R⁵ oder für Wasserstoff oder für einen Alkyl-, Cycloalkyloder Aralkyl-Rest mit 1 bis 12 C-Atomen steht,
R⁴ und R⁵ unabhängig voneinander jeweils für Wasserstoff oder für einen Alkyl-, Cycloalkyl- oder Aralkyl-Rest mit 1 bis 18 C-Atomen, welcher gegebenenfalls Heteroatome enthält, stehen,
wobei
R¹ und R² auch zusammen für einen Alkylen-Rest mit 2 bis 6 C-Atomen stehen können,
R² und R⁰ auch zusammen für einen Alkylen-Rest mit 3 bis 6 C-Atomen, welcher gegebenenfalls Heteroatome enthält, stehen können, R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Heteroatome enthält, stehen können, und
R² und R⁵ auch zusammen für einen Alkylen-Rest mit 2 bis 12 C-Atomen stehen können;
wobei der Katalysator der Formel (I) kein Stickstoffatom enthält, welches direkt an einen aromatischen Ring gebunden oder Teil eines heteroaromatischen Ringsystems ist.

2. Katalysator nach Anspruch 1, **dadurch gekennzeichnet, dass** A für einen Polyoxypropylen-Rest, gegebenenfalls mit Anteilen anderer Oxyalkylen-Einheiten, oder für einen Poly(oxytetramethylen)-Rest, gebenenfalls mit Anteilen von 1,2-Oxypropylen-Einheiten, oder für einen ein- bis dreiwertigen Rest einer polyoxyalkylierten Verbindung aus der Polyaddition von 1,2-Propylenoxid an Startermoleküle ausgewählt aus der Gruppe bestehend aus Alkoholen, Fettalkoholen, Alkylphenolen, Diolen und Triolen steht.

3. Katalysator nach einem der vorhergehenden Ansprüche, **dadurch gekennzeichnet, dass** R³ für -NR⁴R⁵ steht,
R⁴ für Wasserstoff steht,
R² und R⁵ unabhängig voneinander jeweils für einen Alkyl-, Cycloalkyloder Aralkyl-Rest mit 1 bis 18 C-Atomen, welcher gegebenenfalls Heteroatome enthält, stehen, und
R¹ für Wasserstoff steht.

4. Katalysator nach Anspruch 3, **dadurch gekennzeichnet, dass** R² und R⁵ unabhängig voneinander jeweils für Ethyl, Isopropyl, tert.Butyl, 3-(Dimethylamino)propyl oder Cyclohexyl stehen.

5. Verfahren zur Herstellung eines Katalysators nach einem der Ansprüche 1 oder 2 durch die Umsetzung von mindestens einem Polyetheramin mit mindestens einem Reagens zur Einführung einer Amidin- oder Guanidin-Gruppe ausgewählt aus der Gruppe bestehend aus Orthoestern, 1,3-Ketoestern, 1,3-Ketoamiden, Nitrilen, Imidsäureestern, Imidsäurechloriden, Amiden, Lactamen, Cyanamid, Carbodiimiden, Harnstoffen, O-Alkylisoharnstoffen, Thioharnstoffen, S-Alkylisothioharnstoffe, Aminoiminomethansulfonsäuren, Guanylpyrazolen und Guanidinen.

6. Verwendung eines Katalysators nach einem der Ansprüche 1 bis 4 in einer härtbaren Zusammensetzung als Vernetzungskatalysator.

7. Verwendung nach Anspruch 6, dadurch gekennzeichent, dass die härtbare Zusammensetzung einen Klebstoff, einen Dichtstoff oder eine Beschichtung darstellt.

8. Härtbare Zusammensetzung umfassend mindestens einen Katalysator nach einem der Ansprüche 1 bis 4.

9. Härtbare Zusammensetzung nach Anspruch 8, **dadurch gekennzeichnet, dass** sie mindestens ein Silangruppen-haltiges Polymer enthält.

10. Härtbare Zusammensetzung nach Anspruch 9, **dadurch gekennzeichnet, dass** das Silangruppen-haltige Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere ist.

11. Härtbare Zusammensetzung nach einem der Ansprüche 8 bis 10, **dadurch gekennzeichnet, dass** zusätzlich mindestens ein Organotitanat enthalten ist.

12. Ausgehärtete Zusammensetzung, erhalten aus einer härtbaren Zusammensetzung nach einem der Ansprüche 8 bis 11 nach deren Härtung.

## Claims

1. Catalyst of the formula (I),
A-[-Z]ₙ (I)
where
n is 1 or 2 or 3,
A is a polyoxyalkylene radical from the polyaddition of oxiranes, in particular ethylene oxide and/or 1,2-propylene oxide and/or 1,2-butylene oxide, to starter molecules having two active hydrogen atoms, in particular water, glycols, such as ethane-1,2-diol, propane-1,2-diol or -1,3-diol, 2-methylpropane-1,3-diol, butane-1,2-diol, -1,3-diol or -1,4-diol, pentane-1,3-diol or -1,5-diol, 3-methylpentane-1,5-diol, hexane-1,2-diol, -2,5-diol or -1,6-diol, neopentyl glycol, diethylene glycol, triethylene glycol, polyethylene glycols, dipropylene glycol, tripropylene glycol, polypropylene glycols or poly(tetramethylene ether) glycols or a poly(oxytetramethylene) radical from the ring-opening polymerization of tetrahydrofuran, which optionally includes proportions of other oxyalkylene units, such as in particular 1,2-oxypropylene or oxybutylene units; or is a radical of a polyoxyalkylated compound from the polyaddition of ethylene oxide and/or 1,2-propylene oxide and/or 1,2-butylene oxide to starter molecules having one to three active hydrogen atoms, selected from methanol, ethanol, isopropanol, butanol, 2-ethylhexanol, lauryl alcohol, stearyl alcohol, fatty alcohol mixtures from natural fats, phenol, alkylphenols, diols, diphenols, and triols, where A has a molecular weight within a range from 200 to 5000 g/mol,
and optionally has one or two terminal primary or secondary amino groups or hydroxyl groups, and Z is a radical of the formula 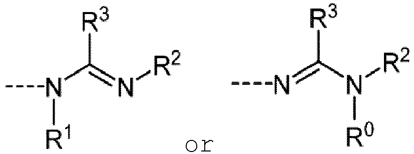 ,
where
R¹ and R⁰ are each independently hydrogen or an alkyl or cycloalkyl or aralkyl radical having 1 to 8 carbon atoms,
R² is hydrogen or an alkyl, cycloalkyl or aralkyl radical having 1 to 18 carbon atoms, which optionally contains heteroatoms and which optionally has terminal primary or secondary amino groups,
R³ is -NR⁴R⁵ or hydrogen or an alkyl, cycloalkyl or aralkyl radical having 1 to 12 carbon atoms, R⁴ and R⁵ are each independently hydrogen or an alkyl, cycloalkyl or aralkyl radical having 1 to 18 carbon atoms, which optionally contains heteroatoms,
where
R¹ and R² may also together be an alkylene radical having 2 to 6 carbon atoms,
R² and R⁰ may also together be an alkylene radical having 3 to 6 carbon atoms, which optionally contains heteroatoms,
R² and R³ may also together be an alkylene radical having 3 to 6 carbon atoms,
R⁴ and R⁵ may also together be an alkylene radical having 4 to 7 carbon atoms, which optionally contains heteroatoms,
and
R² and R⁵ may also together be an alkylene radical having 2 to 12 carbon atoms;
wherein the catalyst of the formula (I) does not contain any nitrogen atom that is directly attached to an aromatic ring or part of a heteroaromatic ring system.

2. Catalyst according to Claim 1, **characterized in that** A is a polyoxypropylene radical, optionally containing proportions of other oxyalkylene units, or is a poly(oxytetramethylene) radical, optionally containing proportions of 1,2-oxypropylene units, or is a monovalent to trivalent radical of a polyoxyalkylated compound from the polyaddition of 1,2-propylene oxide to starter molecules selected from the group consisting of alcohols, fatty alcohols, alkylphenols, diols and triols.

3. Catalyst according to either of the preceding claims, **characterized in that** R³ is -NR⁴R⁵,
R⁴ is hydrogen,
R² and R⁵ are each independently an alkyl, cycloalkyl or aralkyl radical having 1 to 18 carbon atoms, which optionally contains heteroatoms, and
R¹ is hydrogen.

4. Catalyst according to Claim 3, **characterized in that** R² and R⁵ are each independently ethyl, isopropyl, tert-butyl, 3-(dimethylamino)propyl or cyclohexyl.

5. Process for preparing a catalyst according to either of Claims 1 or 2 by the reaction of at least one polyether amine with at least one reagent for introducing an amidine or guanidine group, selected from the group consisting of orthoesters, 1,3-ketoesters, 1,3-ketoamides, nitriles, imidates, imidoyl chlorides, amides, lactams, cyanamide, carbodiimides, ureas, O-alkyl isoureas, thioureas, S-alkyl isothioureas, aminoiminomethanesulfonic acids, guanylpyrazoles and guanidines.

6. Use of a catalyst according to any of Claims 1 to 4 in a curable composition as a crosslinking catalyst.

7. Use according to Claim 6, **characterized in that** the curable composition constitutes an adhesive, a sealant or a coating.

8. Curable composition comprising at least one catalyst according to any of Claims 1 to 4.

9. Curable composition according to Claim 8, **characterized in that** it comprises at least one polymer containing silane groups.

10. Curable composition according to Claim 9, **characterized in that** the polymer containing silane groups is a polyolefin containing silane groups or a polyester containing silane groups or a poly(meth)acrylate containing silane groups or a polyether containing silane groups or a hybrid of said polymers.

11. Curable composition according to any of Claims 8 to 10, **characterized in that** it additionally comprises at least one organotitanate.

12. Cured composition obtained from a curable composition according to any of Claims 8 to 11 after the curing thereof.

## Revendications

1. Catalyseur de formule (I),
A-[-Z]ₙ (I)
dans laquelle
n représente 1 ou 2 ou 3,
A représente un radical polyoxyalkylène provenant de la polyaddition d'oxiranes, en particulier d'oxyde d'éthylène et/ou de 1,2-oxypropylène et/ou de 1,2-oxybutylène sur des molécules de départ comportant deux atomes d'hydrogène actif, en particulier l'eau, des glycols tels que le 1,2-éthanediol le 1,2- ou 1,3-propanediol, le 2-méthyl-1,3-propanediol, le 1,2- ou 1,3- ou 1,4-butanediol, le 1,3- ou 1,5-pentanediol, le 3-méthyl-1,5-pentanediol, le 1,2- ou 2,5- ou 1,6-hexanediol, le néopentylglycol, le diéthylèneglycol, le triéthylèneglycol, les polyéthylèneglycols, le dipropylèneglycol, le tripropylèneglycol, les polypropylèneglycols ou poly(tétraméthylène-éther)-glycols ou un radical poly(oxytétraméthylène) provenant de la polymérisation avec ouverture de cycle du tétrahydrofurane, qui comporte éventuellement des fractions d'autres unités oxyalkylène telles qu'en particulier des unités 1,2-oxypropylène ou oxybutylène ; ou un radical d'un composé polyoxyalkylé provenant de la polyaddition d'oxyde d'éthylène et/ou de 1,2-oxypropylène et/ou de 1,2-oxybutylène sur des molécules de départ comportant de un à trois atomes d'hydrogène actif, choisies parmi le méthanol, l'éthanol, l'isopropanol, le butanol, le 2-éthylhexanol, l'alcool laurylique, l'alcool stéarylique, des mélanges d'alcools gras provenant de graisses naturelles, le phénol, des alkylphénols, des diols, des diphénols et des triols, A présentant une masse moléculaire dans la plage de 200 à 5 000 g/mole,
et comporte éventuellement un ou deux groupes hydroxy ou groupes amino primaires ou secondaires en bout de chaîne, et
Z représente un radical de formule 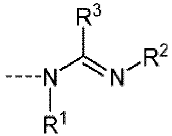 ou 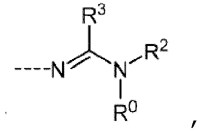 où
R¹ et R⁰ représentent chacun indépendamment l'un de l'autre un atome d'hydrogène ou un radical alkyle ou cycloalkyle ou aralkyle ayant de 1 à 8 atomes de carbone,
R² représente un atome d'hydrogène ou un radical alkyle, cycloalkyle ou aralkyle ayant de 1 à 18 atomes de carbone, qui contient éventuellement des hétéroatomes et qui comporte éventuellement des groupes amino primaires ou secondaires en bout de chaîne,
R³ représente -NR⁴R⁵ ou un atome d'hydrogène ou un radical alkyle, cycloalkyle ou aralkyle ayant de 1 à 12 atomes de carbone,
R⁴ et R⁵ représentent chacun indépendamment l'un de l'autre un atome d'hydrogène ou un radical alkyle, cycloalkyle ou aralkyle ayant de 1 à 18 atomes de carbone, qui contient éventuellement des hétéroatomes, R¹ et R² pouvant également représenter ensemble un radical alkylène ayant de 2 à 6 atomes de carbone,
R² et R⁰ pouvant également représenter ensemble un radical alkylène ayant de 3 à 6 atomes de carbone, qui contient éventuellement des hétéroatomes,
R² et R³ pouvant également représenter ensemble un radical alkylène ayant de 3 à 6 atomes de carbone,
R⁴ et R⁵ pouvant également représenter ensemble un radical alkylène ayant de 4 à 7 atomes de carbone, qui contient éventuellement des hétéroatomes,
et
R² et R⁵ pouvant également représenter ensemble un radical alkylène ayant de 2 à 12 atomes de carbone,
le catalyseur de formule (I) ne contenant pas d'atome d'azote qui est lié directement à un cycle aromatique ou fait partie d'un système cyclique hétéroaromatique.

2. Catalyseur selon la revendication 1, **caractérisé en ce que** A représente un radical polyoxypropylène, éventuellement avec des fractions d'autres unités oxyalkylène, ou un radical poly(oxytétraméthylène), éventuellement avec des fractions d'unités 1,2-oxypropylène, ou un radical mono- à trivalent d'un composé polyoxyalkylé provenant de la polyaddition de 1,2-oxypropylène sur des molécules de départ choisies dans le groupe constitué par les alcools, alcools gras, alkylphénols, diols et triols.

3. Catalyseur selon l'une quelconque des revendications précédentes, **caractérisé en ce que** R³ représente -NR⁴R⁵,
R⁴ représente un atome d'hydrogène,
R² et R⁵ représentent chacun indépendamment l'un de l'autre un radical alkyle, cycloalkyle ou aralkyle ayant de 1 à 18 atomes de carbone, qui contient éventuellement des hétéroatomes, et
R¹ représente un atome d'hydrogène.

4. Catalyseur selon la revendication 3, **caractérisé en ce que** R² et R⁵ représentent chacun indépendamment l'un de l'autre un groupe éthyle, isopropyle, tertbutyle, 3-(diméthylamino)propyle ou cyclohexyle.

5. Procédé pour la préparation d'un catalyseur selon l'une quelconque des revendications 1 et 2, par la mise en réaction d'au moins une polyétheramine avec au moins un partenaire réactionnel destiné à l'introduction d'un groupe amidino ou guanidino, choisi dans le groupe constitué par les orthoesters, 1,3-cétoesters, 1,3-cétoamides, nitriles, esters d'acides imidiques, chlorures d'acides imidiques, amides, lactames, le cyanamide, les carbodiimides, urées, O-alkylisourées, thiourées, S-alkylisothiourées, acides aminoiminométhanesulfoniques, guanylpyrazoles et guanidines.

6. Utilisation d'un catalyseur selon l'une quelconque des revendications 1 à 4 dans une composition durcissable, en tant que catalyseur de réticulation.

7. Utilisation selon la revendication 6, **caractérisée en ce que** la composition durcissable est un adhésif, une matière d'étanchéité ou un revêtement.

8. Composition durcissable comprenant au moins un catalyseur selon l'une quelconque des revendications 1 à 4.

9. Composition durcissable selon la revendication 8, **caractérisée en ce qu'**elle contient au moins un polymère contenant des groupes silane.

10. Composition durcissable selon la revendication 9, **caractérisée en ce que** le polymère contenant des groupes silane est une polyoléfine contenant des groupes silane ou un polyester contenant des groupes silane ou un poly(méth)acrylate contenant des groupes silane ou un polyéther contenant des groupes silane ou une forme mixte de ces polymères.

11. Composition durcissable selon l'une quelconque des revendications 8 à 10, **caractérisée en ce qu'**en outre au moins un organotitanate est contenu.

12. Composition durcie, obtenue à partir d'une composition durcissable selon l'une quelconque des revendications 8 à 11 après son durcissement.
